(19) Europäisches Patentamt
European Patent Office
Office européen des brevets

(11) EP 1 674 454 A1

(12) **EUROPEAN PATENT APPLICATION**
published in accordance with Art. 158(3) EPC

(43) Date of publication:
**28.06.2006 Bulletin 2006/26**

(21) Application number: **04792462.6**

(22) Date of filing: **15.10.2004**

(51) Int Cl.:
***C07D 201/00*** *(1974.07)*

(86) International application number:
**PCT/JP2004/015245**

(87) International publication number:
**WO 2005/037837 (28.04.2005 Gazette 2005/17)**

(84) Designated Contracting States:
**AT BE BG CH CY CZ DE DK EE ES FI FR GB GR HU IE IT LI LU MC NL PL PT RO SE SI SK TR**

(30) Priority: **17.10.2003 JP 2003357143**

(71) Applicant: **NIPPON KAYAKU KABUSHIKI KAISHA**
**Tokyo 102-8172 (JP)**

(72) Inventors:
- **KURAMOCHI, Hiroshi**
  **Minamisaitama-gun, Saitama 3490217 (JP)**
- **MASUDA, Akira**
  **Saitama-shi,**
  **Saitama 3380001 (JP)**
- **SHIMIZU, Kazuhisa**
  **Maebashi-shi,**
  **Gunma 3710823 (JP)**
- **TOYODA, Eriko**
  **Kawasaki-shi, Kanagawa 2150011 (JP)**
- **TOKUNAKA, Kazuhiro**
  **Saitama-shi, Saitama 3300835 (JP)**

(74) Representative: **Gille Hrabal Struck Neidlein Prop Roos**
**Brucknerstrasse 20**
**40593 Düsseldorf (DE)**

# (54) SUBSTITUTED 2-AMINO- 1,2,4 TRIAZOLO 1,5-a PYRIMIDINE DERIVATIVE AND USE THEREOF

(57) Disclosed is a [1,2,4]triazolo[1,5-a]pyrimidine derivative represented by the general formula (I) below or a pharmaceutically acceptable salt thereof. Also disclosed is a medicine containing such a derivative or a salt thereof as an active constituent.

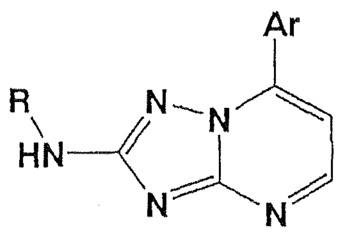

(I)

In the formula, Ar represents a phenyl group which may have a substituent or a 5- or 6-membered aromatic heterocyclic ring which contains one heteroatom and may have a substituent, and R represents a ($C_1$-$C_6$) alkyl group which may be substituted or the like.

EP 1 674 454 A1

**Description**

TECHNICAL FIELD

The present invention relates to an immunosuppresant or an immunotolerance inducer. More specifically, the present invention relates to a drug containing a substituted 2-amino-[1,2,4]triazolo[1,5-a]pyrimidine derivative which can be used for the treatment or prevention of autoimmune diseases, allergic diseases, diseases associated with tissue inflammation, rejection against organ transplantation or bone marrow transplantation or graft versus host reaction as an immunosuppresant. The present invention also relates to a drug containing a substituted 2-amino-[1,2,4]triazolo[1,5-a]pyrimidine derivative which can be used for successfully engrafting an organ or bone marrow transplanted to a patient as an immunotolerance inducer.

BACKGROUND ART

Presently, immunosuppresants, for example, steroid drugs, ciclosporin A, tacrolimus, mycophenolate mofetil, mizoribine, deoxyspergualin, etc. are used for the treatment and prevention of transplantation rejection, autoimmune diseases, allergic diseases and various autoimmune diseases, etc.

It has come to be known in late years that a steroid drug which has been used as an antiinflammatory agent for a long time acts on macrophages and lymphocytes by massive dose and shows immune inhibition action.

Ciclosporin A and tacrolimus inhibit the production of cytokine which is a regulator of lymphocyte and thereby show immune inhibition action. Ciclosporin A is administered for inhibiting transplantation rejection against kidney transplantation, liver transplantation, bone marrow transplantation and heart transplantation, Behcet's disease, psoriasis, aplastic anemia, nephrotic syndrome. Tacrolimus is administered for inhibiting transplantation rejection against kidney transplantation, liver transplantation, bone marrow transplantation and heart transplantation, atopic dermatitis, myasthenia gravis as a stronger cytokine production inhibitant.

Mycophenolate mofetil and mizoribine show immune inhibition action through antagonistic action on nucleic-acid metabolism of lymphocyte. Mycophenolate mofetil is used for inhibiting rejection against kidney transplantation, and mizoribine is used for nephrotic syndrome, lupus nephritis, chronic rheumatoid arthritis.

Deoxyspergualin shows immune inhibition action by inhibiting antibody production and the function of lymphocyte, and is used for the treatment of rejection after kidney transplantation.

Immunosuppresant is also effective for autoimmune diseases other than the diseases mentioned above, and for example, ciclosporin A has been reported to be effective for diseases such as atopic dermatosis, autoimmune hepatitis, Crohn's disease, colitis ulcerosa, myasthenia gravis, multiple sclerosis, rheumatoid arthritis, insulin-dependent diabetes besides the adapted diseases.

In the meantime, immunological phenomenon harmful to the self occurs through presentation of an antigen which causes pathological conditions in the diseases mentioned above. For example, as for autoimmune diseases, it is considered that an autoantigen or a foreign antigen similar to an autoantigen is presented to immunocompetent cells by a dendritic cell, one of the antigen presenting cells, and thereby immune response to the autoantigen is caused and destruction of self-tissues occurs (Non-Patent Document 1).

Accumulation of dendritic cells which are antigen presenting cells is also recognized at lesioned arthrosis part of a patient in rheumatism, an inflammatory disease, and it is considered that presentation of an antigen participates in the onset and aggravation thereof (Non-Patent Document 2).

In addition, when T cell recognizes a cell developing a target antigen, it is performed through MHC (major histocompatibiliy complex), and therefore presentation of an antigen is also considered to participate in activation of T cells and tissue damage at lesioned site in autoimmune diseases and inflammatory diseases (Non-Patent Document 3).

Furthermore, it has been reported that immune tolerance is induced by differences of maturation phase among antigen-presenting dendritic cells. It is considered that matured dendritic cells induce effector T lymphocytes having cytotoxic activity and cytokine production activity whereas immature dendritic cells induce regulatory or inhibitor T cells and have a big role on induction and maintenance of immune tolerance (Non-Patent Document 4).

Synthesis of 2-amino-7-phenyl-[1,2,4]triazolo[1,5-a]pyrimidine substituted with an N-alkenyl group is described in Non-Patent Document 5 but neither 2-amino-7-phenyl-[1,2,4]triazolo[1,5-a]pyrimidine substituted with an N-alkyl group nor a drug using the same is described.

In addition, 2-amino-7-phenyl-[1,2,4]triazolo[1,5-a]pyrimidine substituted with an alkyl group substituted with an amino group which may be substituted is described in Patent Document 1.

Non-Patent Document 1: Ludewig, B. et al., Current Opinion in Immunology, vol. 13, p. 657 (2001))
Non-Patent Document 2: Thomas, R. et al., Journal of Leukocytes Biology, vol. 66, p. 286 (1999))

Non-Patent Document 3: Immunology Illustrated (the fifth edition), Roitt, I. et al. translated under supervision of Tomio Tada, Nankodo (2000), p. 128-131 and p. 355-358
Non-Patent Document 4: Ralph, M.S. et al., Proceedings of the National Academy of Sciences, vol. 99, p. 351 (2002)
Non-Patent Document 5: Allen, C.F.H. et al., J. Org. Chem., 24, p. 796-801 (1959)
Patent Document 1: WO 02/20495

DISCLOSURE OF THE INVENTION

Problems to be Solved by The Invention

Presentation of an antigen by antigen presenting cells which causes pathological conditions is involved in autoimmune diseases, allergic diseases, tissue inflammatory diseases and rejection against organ transplantation or bone marrow transplantation, and it is considered that aberration or excessive immune response can be inhibited by inhibiting the expression of antigen presentation molecules or modifying the antigen presentation by the antigen presenting cells, but a compound having such an action is not known at present.
Antigen presentation is a function specific to immune system, and it is considered that substances which specifically inhibit antigen presentation inhibition/modification action do not exhibit actions other than the immune system, that is, side effects recognized in presently known immunosuppresants.
It is also considered that when maturation of dendritic cells which show an antigen is inhibited, the number of immature dendritic cells increases and immune tolerance is induced, but a compound having such an action is known at present.
An object of the present invention is to provide an immunosuppresant or an immunotolerance inducer having little side effect which inhibits aberration or excessive immune response by inhibiting or modifying the antigen presentation.

MEANS FOR SOLVING PROBLEMS

The present inventors have conducted intensive studies to solve the above-mentioned problems and have found that substituted 2-amino-[1,2,4]triazolo[1,5-a]pyrimidine derivatives inhibit antigen presentation by antigen presenting cells and have immune inhibition action and that the compound inhibits the lymphocyte proliferation response and can be applied as a therapeutic agent or a prophylactic agent for immune disorders, and have completed the present invention.
In addition, the present inventors have found that the compound inhibits the expression of the antigen presentation conjugated molecule which participates in antigen presentation and that it can be used as an immunotolerance inducer and have completed the present invention. The present inventors have also found that the compound has cell proliferation inhibitory activity on cells of lymphoma cell line and that it can be used as an antineoplastic drug and have completed the present invention.
That is, the present invention relate to a drug comprising as an active ingredient a substituted 2-amino-[1,2,4] triazolo[1,5-a]pyrimidine derivative represented by the general formula (I):
[Formula 1]

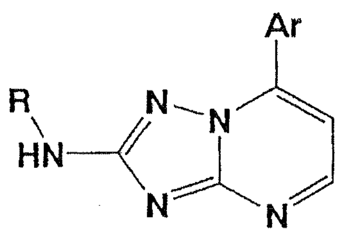

(I)

wherein Ar represents a phenyl group which may have a substituent or a 5- or 6-membered aromatic heterocyclic group which contains one hetero atom and may have a substituent; and
R represents a (C1-C6) alkyl group which may be substituted with a substituent selected from the substituent group consisting of a halogeno group, a cyano group, a nitro group, a hydroxyl group, a (C1-C6) alkoxyl group, a benzyloxy group, a phenyl group and a 5- or 6-membered aromatic heterocyclic group which contains one hetero atom, a (C2-C7) alkoxycarbonyl group which may be substituted with a substituent, a (C1-C13) aliphatic acyl group which may have 1

to 3 same or different substituents, an amino acid group in which the N-terminal may be protected or a 3- to 7-membered cyclic acyl group which may have 1 to 3 same or different substituents, or a pharmaceutically acceptable salt thereof.

Furthermore, the present invention relates to an antigen presentation inhibitor comprising as an active ingredient a substituted 2-amino-[1,2,4]triazolo[1,5-a]pyrimidine derivative or a pharmaceutically acceptable salt thereof mentioned above.

Furthermore, the present invention relates to an immunosuppresant or an immunotolerance inducer comprising as an active ingredient a substituted 2-amino-[1,2,4]triazolo[1,5-a]pyrimidine derivative or a pharmaceutically acceptable salt thereof mentioned above.

Furthermore, the present invention relates to a therapeutic agent or a prophylactic agent for transplant rejection reaction or graft versus host reaction diseases comprising as an active ingredient a substituted 2-amino-[1,2,4]triazolo[1,5-a] pyrimidine derivative or a pharmaceutically acceptable salt thereof mentioned above.

Furthermore, the present invention relates to a therapeutic agent or a prophylactic agent for autoimmune diseases comprising as an active ingredient a substituted 2-amino-[1,2,4]triazolo[1,5-a]pyrimidine derivative or a pharmaceutically acceptable salt thereof mentioned above.

Furthermore, the present invention relates to a therapeutic agent or a prophylactic agent for rheumatoid arthritis, multiple sclerosis, systemic lupus erythematosus, discoid lupus erythematosus, Sjogren's syndrome, Crohn's disease, colitis ulcerosa, idiopathic thrombocytopenia, aplastic anemia, autoimmune hepatitis, insulin-dependent diabetes, myasthenia gravis, polymyositis, scleroderma, mixed connective tissue disease, ankylosing spondylitis or chronic thyroiditis comprising as an active ingredient a substituted 2-amino-[1,2,4]triazolo[1,5-a]pyrimidine derivative or a pharmaceutically acceptable salt thereof mentioned above.

Furthermore, the present invention relates to a therapeutic agent or a prophylactic agent for allergic diseases comprising as an active ingredient a substituted 2-amino-[1,2,4]triazolo[1,5-a]pyrimidine derivative or a pharmaceutically acceptable salt thereof mentioned above.

Furthermore, the present invention relates to a therapeutic agent or a prophylactic agent for atopic dermatosis, pollinosis, contact hypersensitivity, asthma, psoriasis or anaphylaxis comprising as an active ingredient a substituted 2-amino-[1,2,4] triazolo[1,5-a]pyrimidine derivative or a pharmaceutically acceptable salt thereof mentioned above.

Furthermore, the present invention relates to a therapeutic agent or a prophylactic agent for inflammatory diseases comprising as an active ingredient a substituted 2-amino-[1,2,4]triazolo[1,5-a]pyrimidine derivative or a pharmaceutically acceptable salt thereof mentioned above.

Furthermore, the present invention relates to a therapeutic agent or a prophylactic agent for Behcet's disease, polyarteritis, sarcoidosis, glomerulonephritis, nephrotic syndrome, intractable vasculitis or Wegener's syndrome comprising as an active ingredient a substituted 2-amino-[1,2,4]triazolo[1,5-a]pyrimidine derivative or a pharmaceutically acceptable salt thereof mentioned above.

Furthermore, the present invention relates to a cell proliferation inhibitor comprising as an active ingredient a substituted 2-amino-[1,2,4]triazolo[1,5-a]pyrimidine derivative or a pharmaceutically acceptable salt thereof mentioned above.

Furthermore, the present invention relates to an antineoplastic drug comprising as an active ingredient a substituted 2-amino-[1,2,4]triazolo[1,5-a]pyrimidine derivative or a pharmaceutically acceptable salt thereof mentioned above.

Furthermore, the present invention relate to a substituted 2-amino-[1,2,4]triazolo[1,5-a]pyrimidine derivative represented by the general formula (III):

[Formula 2]

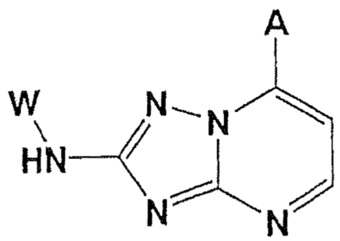

(III)

wherein A represents a phenyl group which may be substituted with 1 to 3 same or different substituents selected from the substituent group (A) consisting of a halogeno group, a hydroxyl group, a (C1-C6) alkyl group, a cyano group, a nitro group, a (C1-C6) alkoxyl group, a benzyloxy group, an amino group, a (C1-C7) acylamino group and a methylenedioxy group or a 5- or 6-membered aromatic heterocyclic group which contains one hetero atom and may be

substituted with 1 to 3 same or different substituents selected from the substituent group (A); and
W represents a (C1-C6) alkyl group which may be substituted with one substituent selected from the substituent group consisting of a halogeno group, a hydroxyl group, a (C1-C6) alkoxyl group, a phenyl group and a 5- or 6-membered aromatic heterocyclic group which contains one hetero atom;
a (C2-C7) alkoxycarbonyl group which may be substituted with one substituent selected from the substituent group consisting of a phenyl group, a methoxyphenyl group and a 5- or 6-membered aromatic heterocyclic group which contains one hetero atom;
a (C1-C13) aliphatic acyl group which may have 1 to 3 same or different substituents selected from the substituent group consisting of a halogeno group, a hydroxyl group, an oxo group, a (C1-C6) alkoxyl group, a (C1-C7) acyl group, a (C1-C7) acyloxy group, a trifluoromethyl group, a trifluoromethoxy group, a cyano group, a nitro group, a (C1-C6) alkylsulfanyl group, a benzylsulfanyl group, an arylsulfanyl group, a (C1-C6) alkylsulfonyl group, an arylsulfonyl group, a (C1-C6) alkoxycarbonyl group, an amino group, a (C1-C6) alkylamino group, a di(C1-C6) alkylamino group, a (C1-C7) acylamino group, a (C1-C6) alkoxycarbonylamino group, a benzyloxycarbonylamino group, a phenyl group, wherein the phenyl group may be substituted with 1 to 3 substituents selected from the substituent group consisting of a halogeno group, a (C1-C6) alkyl group, a trifluoromethyl group, a cyano group, a nitro group, a (C1-C6) alkoxyl group, a benzyloxy group, an amino group, a di(C1-C6) alkylamino group, or a 5- or 6-membered saturated or unsaturated heterocyclic group which contains 1 to 4 hetero atoms independently selected from N, O and S and may be substituted with 1 to 3 (C1-C6) alkyl groups;
an α-amino acid group which may be protected at the N-terminal; or
a cyclic acyl group represented by Z-CO-, wherein Z represents an aromatic hydrocarbon group which may have 1 to 3 same or different substituents selected from the substituent group (B) consisting of a (C1-C6) alkyl group, a halogeno group, a hydroxyl group, an oxo group, a trifluoromethyl group, a cyano group, a nitro group, a (C1-C6) alkoxyl group, a benzyloxy group, a (C1-C6) alkylsulfanyl group, a (C1-C6) alkoxycarbonyl group, an amino group, a di(C1-C6) alkylamino group, a (C1-C7) acylamino group and a methylenedioxy group, or a 5- or 6-membered saturated or unsaturated heterocyclic group which contains 1 to 4 hetero atoms independently selected from N, O and S and may be substituted with 1 to 3 same or different substituents selected from the substituent group (B), or a pharmaceutically acceptable salt thereof.

Furthermore, the present invention relates to a substituted 2-amino-[1,2,4]triazolo[1,5-a]pyrimidine derivative which is 3-benzenesulfonyl-N-(7-thiophen-2-yl-[1,2,4]triazolo[1,5-a]pyrimidin-2-yl)propionamide, 3-benzenesulfonyl-N-(7-(4-methoxyphenyl)-[1,2,4]triazolo[1,5-a]pyrimidin-2-yl)propionamide, (S)-2-(tert-butoxycarbonyl)amino-N-(7-thiophen-2-yl-[1,2,4]triazolo[1,5-a]pyrimidin-2-yl)butylamide, (S)-2-amino-N-(7-thiophen-2-yl-[1,2,4]triazolo[1,5-a]pyrimidin-2-yl)butylamide or (S)-2-amino-3-methyl-N-(7-thlophen-2-yl-[1,2,4]triazolo[1,5-a]pyrimidin-2-yl)butylamide, or a pharmaceutically acceptable salt thereof.

## EFFECTS OF THE INVENTION

The substituted 2-amino-[1,2,4]triazolo[1,5-a]pyrimidin-2-yl urea derivatives represented by general formula (I) of the present invention or pharmaceutically acceptable salts thereof show antigen presentation inhibitory effect and lymphocyte function inhibitory effect and are useful as a drug for the treatment or prevention of autoimmune diseases, transplantation rejection, graft versus host reaction, allergic diseases or inflammatory diseases. In addition, they inhibit expression of a costimulatory molecule participating in antigen presentation and are useful as a drug for immune tolerance induction.

Furthermore, they show excellent activity in in vitro and in vivo immune inhibition activity test and can be used as prophylactic and/or therapeutic drugs for rejection reaction in the transplantation of organs and bone marrow and/or graft versus host reaction, autoimmune diseases, allergic diseases and/or of inflammatory diseases, and an immunotolerance inducer on transplanted organs and transplanted bone marrow.
In addition, they have cell proliferation inhibitory effect and can be used as a drug for the treatment of malignant tumor.

## BEST MODE FOR CARRYING OUT THE INVENTION

The drug of the present invention comprises as an active ingredient a substituted 2-amino-[1,2,4]triazolo[1,5-a]pyrimidine derivative represented by the above general formula (I),
wherein Ar represents a phenyl group which may have a substituent or a 5- or 6-membered aromatic heterocyclic group which contains one hetero atom and may have a substituent; and
R represents a (C1-C6) alkyl group which may be substituted with a substituent selected from the substituent group consisting of a halogeno group, a cyano group, a nitro group, a hydroxyl group, a (C1-C6) alkoxyl group, a benzyloxy group, a phenyl group and a 5- or 6-membered aromatic heterocyclic group which contains one hetero atom, a (C2-C7) alkoxycarbonyl group which may be substituted with a substituent, a (C1-C13) aliphatic acyl group which may have 1

to 3 same or different substituents, an amino acid group in which the N-terminal may be protected or a 3- to 7-membered cyclic acyl group which may have 1 to 3 same or different substituents, or a pharmaceutically acceptable salt thereof.

Preferable substituent Ar in the general formula (I) includes 1-3 same or different groups selected from the substituent group (A) consisting of a halogeno group, a hydroxyl group, a (C1-C6) alkyl group, a cyano group, a nitro group, a (C1-C6) alkoxyl group, a benzyloxyl group, an amino group, a (C1-C7) acylamino group and a methylenedioxy group.

The substituent on (C1-C6) alkyl group in R of general formula (I) includes a substituent selected from the substituent group consisting of a halogeno group, a cyano group, a nitro group, a hydroxyl group, a (C1-C6) alkoxyl group, a benzyloxy group, a phenyl group and a 5- or 6-membered aromatic heterocyclic group which contains one hetero atom, and preferably includes one substituent selected from the substituent group consisting of a halogeno group, a hydroxyl group, a (C1-C6) alkoxyl group, a phenyl group and a 5- or 6-membered aromatic heterocyclic group which contains one hetero atom.

The substituent on (C2-C7) alkoxycarbonyl group in R of general formula (I) preferably includes one substituent selected from the substituent group consisting of a phenyl group, a methoxyphenyl group and a 5- or 6-membered aromatic heterocyclic group which contains one hetero atom.

The substituent on (C1-C13) aliphatic acyl group in R of general formula (I) preferably includes 1-3 same or different groups selected from the substituent group consisting of

a halogeno group, a hydroxyl group, an oxo group, a (C1-C6) alkoxyl group, a (C1-C7) acyl group, a (C1-C7) acyloxy group, a trifluoromethyl group, a trifluoromethoxy group, a cyano group, a nitro group, a (C1-C6) alkylsulfanyl group, a benzylsulfanyl group, an arylsulfanyl group, a (C1-C6) alkylsulfonyl group, an arylsulfonyl group, a (C1-C6) alkoxycarbonyl group, an amino group, a (C1-C6) alkylamino group, a di(C1-C6) alkylamino group, a (C1-C7) acylamino group, a (C1-C6) alkoxycarbonylamino group, a benzyloxycarbonylamino group, a phenyl group, wherein the phenyl group may be substituted with 1 to 3 substituents selected from the substituent group consisting of a halogeno group, a (C1-C6) alkyl group, a trifluoromethyl group, a cyano group, a nitro group, a (C1-C6) alkoxyl group, a benzyloxy group, an amino group, a di(C1-C6) alkylamino group, or a 5- or 6-membered saturated or unsaturated heterocyclic group which contains 1 to 4 hetero atoms independently selected from N, O and S and may be substituted with 1 to 3 (C1-C6) alkyl groups.

The amino acid group in which N-terminal may by protected in R of general formula (I) includes an α-amino acid group in which N-terminal may by protected, and preferably includes an α-amino acid group represented by the following general formula (II):

[Formula 3]

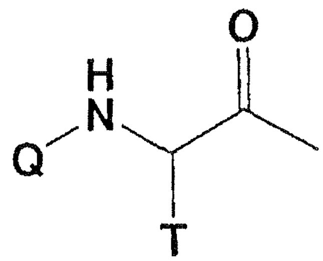

(II)

wherein T represents a hydrogen atom, a methyl group, an ethyl group, a propyl group or an isopropyl group; Q represents a hydrogen atom, a (C1-C7) acyl group or a (C1-C6) alkoxycarbonyl group.

The 3- to 7-membered cyclic acyl group which may have 1 to 3 same or different substituents in R of general formula (I) is preferably Z-CO-, wherein Z represents an aromatic hydrocarbon group which may have a substituent or a 5- or 6-membered saturated or unsaturated heterocyclic group which contains 1 to 4 hetero atoms independently selected from N, O and S and may have a substituent, and the substituent preferably includes 1 to 3 same or different substituents selected from the substituent group (B) consisting of a (C1-C6) alkyl group, a halogeno group, a hydroxyl group, an oxo group, a trifluoromethyl group, a cyano group, a nitro group, a (C1-C6) alkoxyl group, a benzyloxy group, a (C1-C6) alkylsulfanyl group, a (C1-C6) alkoxycarbonyl group, an amino group, a di(C1-C6) alkylamino group, a (C1-C7) acylamino group and a methylenedioxy group.

The 5- or 6-membered aromatic heterocyclic group which contains one hetero atom in Ar of a compound represented by general formula (I) in the drug of the present invention is a 5- or 6-membered aromatic heterocyclic group

containing one hetero atom which is preferably selected from N, O and S independently, and specifically includes a furan-2-yl group, a furan-3-yl group, a thiophen-2-yl group, a thiophen-3-yl group, a pyridin-2-yl group, a pyridin-3-yl group or a pyridin-4-yl group, and preferably it is a thiophen-2-yl group, a thiophen-3-yl group, a pyridin-3-yl group or a pyridin-4-yl group, and more preferably it is a thiophen-2-yl group or a thiophen-3-yl group.

The halogeno group in the present invention is a fluoro group, a chloro group, a bromo group or an iodo group, and preferably it is a fluoro group or a chloro group, and more preferably it is a fluoro group.

The (C1-C6) alkyl group in the present invention refers to a linear, branched chain or cyclic alkyl group having 1 to 6 carbon atoms and includes, for example, a methyl group, an ethyl group, a propyl group, an isopropyl group, a butyl group, an isobutyl group, a tert-butyl group, a pentyl group, an isopentyl group, a 2-methylbutyl group, a neopentyl group, a 1-ethylpropyl group, a hexyl group, a 4-methylpentyl group, a 3-methylpentyl group, a 2-methylpentyl group, a 1-methylpentyl group, a 3,3-dimethylbutyl group, a 2,2-dimethylbutyl group, a 1,1-dimethylbutyl group, a 1,2-dimethylbutyl group, a 1,3-dimethylbutyl group, a 2,3-dimethylbutyl group, a 2-ethylbutyl group, a cyclopropyl group, a cyclopentyl group and a cyclohexyl group, and preferably it is a methyl group, an ethyl group, a propyl group, an isopropyl group, a butyl group, an isobutyl group or a tert-butyl group in, and more preferably it is a methyl group, an ethyl group or an isopropyl group.

The (C1-C6) alkoxy group in the present invention refers to a group in which a (C1-C6) alkyl group as mentioned above is linked with an oxygen atom and includes, for example, a methoxy group, an ethoxy group, a propoxy group, an isopropoxy group, a butoxy group, an isobutoxy group, a tert-butoxy group, a pentyloxy group, an isopentyloxy group, a 2-methylbutoxy group, a neopentyloxy group, a 1-ethylpropoxy group, a hexyloxy group, a 4-methylpentyloxy group, a 3-methylpentyloxy group, a 2-methylpentyloxy group, a 1-methylpentyloxy group, a 3,3-dimethylbutoxy group, a 2,2-dimethylbutoxy group, a 1,1-dimethylbutoxy group, a 1,2-dimethylbutoxy group, a 1,3-dimethylbutoxy group, a 2,3-dimethylbutoxy group, a 2-ethylbutoxy group, a cyclopropoxy group, a cyclopentyloxy group or a cyclohexyloxy group. Preferably it is a methoxy group, an ethoxy group, a propoxy group, an isopropoxy group a butoxy group, an isobutoxy group or a tert-butoxy group, and preferably it is a methoxy group, an ethoxy group or an isopropoxy group.

The (C2-C7) alkoxycarbonyl group in the present invention refers to a group in which a (C1-C6) alkoxyl group as mentioned above is linked with a carbonyl group (C=O) and includes, for example, a methoxycarbonyl group, an ethoxycarbonyl group, a propoxycarbonyl group, an isopropoxycarbonyl group, a butoxycarbonyl group, an isobutoxycarbonyl group, a tert-butoxycarbonyl group, a pentyloxycarbonyl group, an isopentyloxycarbonyl group, a 2-methylbutoxycarbonyl groups, a neopentyloxycarbonyl group, a 1-ethylpropoxycarbonyl group, a hexyloxycarbonyl group, a 4-methylpentyloxycarbonyl group, a 3-methylpentyloxycarbonyl group, a 2-methylpentyloxycarbonyl group, a 1-methylpentyloxycarbonyl group, a 3,3-dimethylbutoxycarbonyl group, a 2,2-dimethylbutoxycarbonyl group, a 1,1-dimethylbutoxycarbonyl group, a 1,2-dimethylbutoxycarbonyl group, a 1,3-dimethylbutoxycarbonyl group, a 2,3-dimethylbutoxycarbonyl group, a 2-ethylbutoxycarbonyl group, a cyclopropoxycarbonyl group, a cyclopentyloxycarbonyl group or a cyclohexyloxycarbonyl group. Preferably it is a methoxycarbonyl group, an ethoxycarbonyl group, a propoxycarbonyl group, an isopropoxycarbonyl group, a butoxycarbonyl group, an isobutoxycarbonyl group or a tert-butoxycarbonyl group, and more preferably it is an ethoxycarbonyl group or a tert-butoxycarbonyl group.

The (C1-C13) acyl group in R of a compound represented by general formula (I) in the drug of the present invention is a group in which a formyl group or a (C1-C12) alkyl group is linked with a carbonyl group (C=O), and the (C1-C12) alkyl group includes a linear, branched or cyclic alkyl group having 1 to 12 carbon atoms which may contain an unsaturated double bond, and, for example, includes a methyl group, an ethyl group, a propyl group, an isopropyl group, a butyl group, an isobutyl group, a tert-butyl group, a pentyl group, an isopentyl group, a 2-methylbutyl group, a neopentyl group, a 1-ethylpropyl group, a hexyl group, a 4-methylpentyl group, a 3-methylpentyl group, a 2-methylpentyl group, a 1-methylpentyl group, a 3,3-dimethylbutyl group, a 2,2-dimethylbutyl group, a 1,1-dimethylbutyl group, a 1,2-dimethylbutyl group, a 1,3-dimethylbutyl group, a 2,3-dimethylbutyl group, a 2-ethylbutyl group, a heptyl group, a 1-methylhexyl group, a 2-methylhexyl group, a 3-methylhexyl groups, a 4-methylhexyl groups, a 5-methylhexyl groups, a 1-propylbutyl group, a 4,4-dimethylpentyl group, an octyl group, a 1-methylheptyl group, a 2-methylheptyl group, a 3-methylheptyl group, a 4-methylheptyl group, a 5-methylheptyl group, a 6-methylheptyl group, a 1-propylpentyl group, a 2-ethylhexyl group, a 5,5-dimethylhexyl group, a nonyl group, a 3-methyloctyl group, a 4-methyloctyl group, a 5-methyloctyl group, a 6-methyloctyl group, a 1-propylhexyl group, a 2-ethylheptyl group, a 6,6-dimethylheptyl group, a decyl group, a 1-methylnonyl group, a 3-methylnonyl group, a 8-methylnonyl group, a 3-ethyloctyl group, a 3,7-dimethyloctyl group, a 7,7-dimethyloctyl group, a decyl group, an undecyl group, a dodecyl group, a cyclopropyl group, a cyclopentyl group, a cyclohexyl group, a 4-methylcyclohexyl group, a 2,6-dimethylcyclohexyl group, a 4,4-dimethylcyclohexyl group, a cycloheptyl group, an adamantan-1-yl group, an adamantan-2-yl group, a 7,7-dimethylbicyclo[2.2.1]heptan-1-yl group, a bicyclo[2.2.1]heptan-2-ylmethyl group, a 4,7,7-trimethylbicyclo[2.2.1] heptan-2-ylmethyl group, a vinyl group, a propenyl group, a 2-methylpropenyl group or a cyclohexan-1-enyl group. Preferably it is a methyl group, an ethyl group, a propyl group, an isopropyl group, a butyl group, an isobutyl group, a tert-butyl group, a pentyl group, an isopentyl group, a neopentyl group, a hexyl group, a 4-methylpentyl group, a 3,3-dimethylbutyl group, a 5-methylhexyl group, a 4,4-dimethylpentyl group, a cyclopentyl group, a cyclohexyl group, a vinyl group, a propenyl group or a 2-methylpropenyl group,

and more preferably it is a methyl group, an ethyl group, a propyl group, a tert-butyl group, an isopentyl group, a 4-methylpentyl group, a vinyl group, a propenyl group or a 2-methylpropenyl group.

The (C1-C7) acyl group in the present invention in an acyl group having 1 to 7 carbon atoms among (C1-C13) acyl groups represented by R of a compound represented by the above general formula (I) and specifically includes, for example, a formyl group, an acetyl group, a propionyl group, a butyryl group, an isobutyryl group, a valeryl group, an isovaleryl group, a pivaloyl group, a hexanoyl group, a cyclopropylcarbonyl group, a cyclopentylcarbonyl group or a cyclohexanecarbonyl group.

The (C1-C7) acyloxy group in the present invention is a group in which a (C1-C7) acyl group mentioned above is linked with an oxygen atom and includes, for example, a formyloxy group, an acetoxy group, a propionyloxy group, a butyryloxy group, an isobutyryloxy group, a valeryloxy group, an isovaleryloxy group, a pivaloyloxy group, a hexanoyloxy group, a cyclopropylcarbonyloxy group, a cyclopentylcarbonyloxy group or a cyclohexanecarbonyloxy group. Preferably it is an acetoxy group, a propionyloxy group or a pivaloyloxy group, and preferably it is an acetoxy group.

The (C1-C7) acylamino group in the present invention refers to a group in which a (C1-C7) acyl group as mentioned above is linked with a nitrogen atom and includes, for example, a formylamino group, an acetylamino group, a propionylamino group, a butyrylamino group, an isobutyrylamino group, a valerylamino group, an isovalerylamino group, a pivaloylamino group, a hexanoylamino group, a cyclopropylcarbonylamino group, a cyclopentylcarbonylamino group or a cyclohexanecarbonylamino group. Preferably it is an acetylamino group, a propionylamino group or a pivaloylamino group, and more preferably it is an acetylamino group.

The (C1-C6) alkylsulfanyl group in the present invention refers to a group in which a (C1-C6) alkyl group as mentioned above is linked with a sulfur atom and includes, for example, a methylsulfanyl group, an ethylsulfanyl group, a propylsulfanyl group, an isopropylsulfanyl group, a butylsulfanyl group, an isobutylsulfanyl group, a tert-butylsulfanyl group, a pentylsulfanyl group, an isopentylsulfanyl group, a 2-methylbutylsulfanyl groups, a neopentylsulfanyl group, a 1-ethylpropylsulfanyl group, a hexylsulfanyl group, a 4-methylpentylsulfanyl group, a 3-methylpentylsulfanyl group, a 2-methylpentylsulfanyl group, a 1-methylpentylsulfanyl group, a 3,3-dimethylbutylsulfanyl group, a 2,2-dimethylbutylsulfanyl group, a 1,1-dimethylbutylsulfanyl group, a 1,2-dimethylbutylsulfanyl group, a 1,3-dimethylbutylsulfanyl group, a 2,3-dimethylbutylsulfanyl group, a 2-ethylbutylsulfanyl group, a cyclopropylsulfanyl group, a cyclopentylsulfanyl group or a cyclohexylsulfanyl group. Preferably it is a methylsulfanyl group, an ethylsulfanyl group, a propylsulfanyl group, an isopropylsulfanyl group, a butylsulfanyl group, an isobutylsulfanyl group or a tert-butylsulfanyl group, and more preferably it is a methylsulfanyl group or an ethylsulfanyl group.

The arylsulfanyl group in the present invention is preferably a group in which an aromatic hydrocarbon group having 6 to 10 carbon atoms is linked with a sulfur atom and includes, for example, a phenylsulfanyl group, a toluen-2-ylsulfanyl group, a toluen-3-ylsulfanyl group, a toluen-4-ylsulfanyl group, a naphthalen-1-ylsulfanyl group or a naphthalen-2-ylsulfanyl group, and preferably it is a phenylsulfanyl group, a toluen-3-ylsulfanyl group or a toluen-4-ylsulfanyl group, and more preferably it is a phenylsulfanyl group.

The (C1-C6) alkylsulfonyl group in the present invention is preferably a group in which a (C1-C6) alkyl group mentioned above is linked with a sulfonyl group and includes, for example, a methylsulfonyl group, an ethylsulfonyl group, a propylsulfonyl group, an isopropylsulfonyl group, a butylsulfonyl group, an isobutylsulfonyl group, a tert-butylsulfonyl group, a pentylsulfonyl group, an isopentylsulfonyl group, a 2-methylbutylsulfonyl group, a neopentylsulfonyl group, a 1-ethylpropylsulfonyl group, a hexylsulfonyl group, a 4-methylpentylsulfonyl group, a 3-methylpentylsulfonyl group, a 2-methylpentylsulfonyl group, a 1-methylpentylsulfonyl group, a 3,3-dimethylbutylsulfonyl group, a 2,2-dimethylbutylsulfonyl group, a 1,1-dimethylbutylsulfonyl group, a 1,2-dimethylbutylsulfonyl group, a 1,3-dimethylbutylsulfonyl group, a 2,3-dimethylbutylsulfonyl group, a 2-ethylbutylsulfonyl group, a cyclopropylsulfonyl group, a cyclopentylsulfonyl group or a cyclohexylsulfonyl group. Preferably it is a methylsulfonyl group, an ethylsulfonyl group, a propylsulfonyl group, an isopropylsulfonyl group, a butylsulfonyl group, an isobutylsulfonyl group or a tert-butylsulfonyl group, and more preferably it is a methylsulfonyl group or an ethylsulfonyl group.

The arylsulfonyl group in the present invention is preferably a group in which an aromatic hydrocarbon having a 6 to 10 carbon atoms is linked with a sulfonyl group and includes, for example, a phenylsulfonyl group, a toluen-2-ylsulfonyl group, a toluen-3-ylsulfonyl group, a toluen-4-ylsulfonyl group, a naphthalen-1-ylsulfonyl group or a naphthalen-2-ylsulfonyl group. Preferably it is a phenylsulfonyl group, a toluen-3-ylsulfonyl group or a toluen-4-ylsulfonyl group, and more preferably it is a phenylsulfonyl group.

The (C1-C6) alkylamino group in the present invention is a group in which one (C1-C6) alkyl group mentioned above is linked with a nitrogen atom and includes, for example, a methylamino group, an ethylamino group, a propylamino group, an isopropylamino group, a butylamino group, an isobutylamino group, a tert-butylamino group, a pentylamino group, an isopentylamino group, a 2-methylbutylamino group, a neopentylamino group, a 1-ethylpropylamino group, a hexylamino group, a 4-methylpentylamino group, a 3-methylpentylamino group, a 2-methylpentylamino group, a 1-methylpentylamino group, a 3,3-dimethylbutylamino group, a 2,2-dimethylbutylamino group, a 1,1-dimethylbutylamino group, a 1,2-dimethylbutylamino group, a 1,3-dimethylbutylamino group, a 2,3-dimethylbutylamino group, a 2-ethylbutylamino group, a cyclopropylamino group, a cyclopentylamino group or a cyclohexylamino group. Preferably it is a

methylamino group, an ethylamino group, a propylamino group, an isopropylamino group, a butylamino group, an isobutylamino group or a tert-butylamino group, and more preferably it is a methylamino group or an ethylamino group.

The di(C1-C6) alkylamino group in the present invention is a group in which two identical (C1-C6) alkyl groups mentioned above are linked with a nitrogen atom and includes, for example, a dimethylamino group, a diethylamino group, a dipropylamino group, a diisopropylamino group, a dibutylamino group, a diisobutylamino group, a dipentylamino group, a dihexylamino group, a bis(3,3-dimethylbutyl)amino group, a dicyclopropylamino group, a dicyclopentylamino group or a dicyclohexylamino group. Preferably it is a dimethylamino group, a diethylamino group, a dipropylamino group, a diisopropylamino group, a dibutylamino group or a diisobutylamino group, and more preferably it is a dimethylamino group or a diethylamino group.

The (C2-C7) alkoxycarbonylamino group in the present invention is a group in which a (C2-C7) alkoxycarbonyl group mentioned above is linked with a nitrogen atom and includes, for example, a methoxycarbonylamino group, an ethoxycarbonylamino group, a propoxycarbonylamino group, an isopropoxycarbonylamino group, a butoxycarbonylamino group, an isobutoxycarbonylamino group, a tert-butoxycarbonylamino group, a pentyloxycarbonylamino group, an isopentyloxycarbonylamino group, a 2-methylbutoxycarbonylamino group, a neopentyloxycarbonylamino group, a 1-ethylpropoxycarbonylamino group, a hexyloxycarbonylamino group, a 4-methylpentyloxycarbonylamino group, a 3-methylpentyloxycarbonylamino group, a 2-methylpentyloxycarbonylamino group, a 1-methylpentyloxycarbonylamino group, a 3,3-dimethylbutoxycarbonylamino group, a 2,2-dimethylbutoxycarbonylamino group, a 1,1-dimethylbutoxycarbonylamino group, a 1,2-dimethylbutoxycarbonylamino group, 1,3-dimethylbutoxycarbonylamino group, 2,3-dimethylbutoxycarbonylamino group, a 2-ethylbutoxycarbonylamino group, a cyclopropoxycarbonylamino group, a cyclopentyloxycarbonylamino group or a cyclohexyloxycarbonylamino group. Preferably it is a methoxycarbonylamino group, an ethoxycarbonylamino group, a propoxycarbonylamino group, an isopropoxycarbonylamino group, a butoxycarbonylamino group, an isobutoxycarbonylamino group or a tert-butoxycarbonylamino group, and more preferably it is an ethoxycarbonylamino group, a tert-butoxycarbonylamino group.

The 5- or 6-membered aromatic heterocyclic group which contains which contains 1 to 4 hetero atoms independently selected from N, O and S as a substituent in R of a compound represented by general formula (I) in the drug of the present invention preferably includes, for example, saturated heterocyclic groups such as a tetrahydrofuran-2-yl group, a tetrahydropyran-2-yl group, a tetrahydropyran-4-yl group, a [1,3]dioxolan-2-yl group, a [1,3]dioxan-2-yl group, a pyrrolidin-1-yl group, a piperidin-1-yl group, a piperidin-4-yl group, an azepan-1-yl group, a morpholin-4-yl group, a thiomorpholin-4-yl group, an oxazolidin-3-yl group, an isoxazolidin-2-yl group, a thiazolidin-3-yl group, an imidazolidin-1-yl group, a piperazin-1-yl group, or saturated heterocyclic groups such as a dihydro-furan-2-yl group, a furan-2-yl group, a furan-3-yl group, a dihydropyran-2-yl group, a thiophen-2-yl group, a thiophen-3-yl group, an oxazol-5-yl group, an isoxazol-5-yl group, a thiazol-5-yl group, a pyrrol-1-yl group, a pyrrol-2-yl group, a pyridin-2-yl group, a pyridin-3-yl group, a pyridin-4-yl group, a pyrimidin-4-yl group, a pyrazin-2-yl group, a [1,3,5]triazin-2-yl group, an imidazol-1-yl group, an imidazol-2-yl group, an imidazol-4-yl group, a [1,2,4]triazol-1-yl group, a [1,2,4]triazol-3-yl group, a tetrazol-1-yl group, a tetrazol-5-yl group.

The above heterocyclic group in a substituent in the case where R is a (C1-C13) aliphatic acyl group in general formula (I) is more preferably a tetrahydrofuran-2-yl group, a tetrahydropyran-4-yl group, a furan-2-yl group, a furan-3-yl group, a thiophen-2-yl group, a thiophen-3-yl group, a pyridin-2-yl group, a pyridin-3-yl group or a pyridin-4-yl group, and a furan-2-yl group, a thiophen-2-yl group or a pyridin-3-yl group is particularly preferable.

The above heterocyclic group in Z in the case where R is a cyclic acyl group Z-CO- in general formula (I) is more preferably unsaturated heterocyclic groups such as a furan-2-yl group, a furan-3-yl group, a dihydropyran-2-yl group, a thiophen-2-yl group, a thiophen-3-yl group, a pyrrol-2-yl group, a pyridin-2-yl group, a pyridin-3-yl group, a pyridin-4-yl group, a pyrimidin-4-yl group or a pyrazin-2-yl group and a furan-2-yl group, a dihydropyran-2-yl group, a thiophen-2-yl group, a pyrrol-2-yl group or a pyridin-3-yl group is particularly preferable.

Specific examples of Ar in a compound represented by general formula (I) in the drug of the present invention includes, for example, a phenyl group, a 3-fluorophenyl group, a 3-chlorophenyl group, a 3-hydroxyphenyl group, a 3-cyanophenyl group, a 3-nitrophenyl group, a 3-methylphenyl group, a 3-ethylphenyl group, a 3-propylphenyl group, a 3-isopropylphenyl group, a 3-butylphenyl group, a 3-methoxyphenyl group, a 3-ethoxyphenyl group, a 3-isopropoxyphenyl group, a 3-isobutoxyphenyl group, a 3-aminophenyl group, a 3-acetylaminophenyl group, a 3-propionylaminophenyl group, a 4-fluorophenyl group, a 4-chlorophenyl group, a 4-hydroxyphenyl group, a 4-cyanophenyl group, a 4-nitrophenyl group, a 4-methylphenyl group, a 4-ethylphenyl group, a 4-propylphenyl group, a 4-isopropylphenyl group, a 4-butylphenyl group, a 4-methoxyphenyl group, a 4-ethoxyphenyl group, a 4-isopropoxyphenyl group, a 4-isobutoxyphenyl group, a 4-aminophenyl group, a 4-acetylaminophenyl group, a 4-propionylaminophenyl group, a 3,4-dihydroxyphenyl group, a 2,4-dimethoxyphenyl group, a 3,4-dimethoxyphenyl group, a 3,4- methylenedioxyphenyl group, a naphthalen-1-yl group, a naphthalen-2-yl group, a furan-2-yl group, a 5-methylfuran-2-yl group, a furan-3-yl group, a thiophen-2-yl group, a 5-methylthiophen-2-yl group, a thiophen-3-yl group, an oxazol-5-yl group, an isoxazol-5-yl group, a thiazol-5-yl group, a pyridin-2 yl group, a pyridin-3-yl group or a pyridin-4-yl group. Preferably it is a phenyl group, a 3-fluorophenyl group, a 3-chlorophenyl group, a 3-methoxyphenyl group, a 4-fluorophenyl group, a 4-chlorophenyl group, a 4-hydroxyphenyl

group, a 4-nitrophenyl group, a 4-methoxyphenyl group, a 4-ethoxyphenyl group, a 4-aminophenyl group, a 4-acetylaminophenyl group, a 4-propionyl aminophenyl group, a 3,4-methylenedioxyphenyl group, a thiophen-2-yl group, a thiophen-3-yl group, a pyridin-2-yl group, a pyridin-3-yl group or a pyridin-4-yl group, and more preferably it is a phenyl group, a 4-methoxyphenyl group or a thiophen-2-yl group.

Specific examples of R in a compound represented by general formula (I) in the drug of the present invention are preferably a methyl group, an ethyl group, a tert-butoxycarbonyl group, an acetyl group, a benzoyl group, a 4-chloro-2-methoxybenzoyl group, a furan-2-ylcarbonyl group, a 3-(phenylsulfonyl)propionyl group, an (S)-2-aminopropionyl group, an (S)-2-aminobutyryl group, an (S)-2-amino-3-methylbutyryl group, an (S)-2-(tert-butoxycarbonyl)aminopropionyl group, an (S)-2-(tert-butoxycarbonyl)aminobutyryl group or an (S)-2-(tert-butoxycarbonyl)amino-3-methylbutyryl group, and preferably a 3-(phenylsulfonyl)propionyl group, an (S)-2-aminobutyryl group, an (S)-2-amino-3-methylbutyryl group or an (S)-2-(tert-butoxycarbonyl)aminobutyryl group.

Therefore, specific compounds of substituted 2-amino-[1,2,4]triazolo[1,5-a]pyrimidine derivatives represented by general formula (I) in a drug of the present invention correspond to compounds derived by combining specific examples of Ar and R mentioned above. When they have isomers, each isomer and a mixture thereof are included in the present invention. That is, in the case of optical isomer, racemic compound, each enantiomer and a mixture thereof in any ratio are also included in the present invention. Such optical isomers are prepared by a conventional production process, that is, synthesis using optical active raw materials or optical resolution method or separation method of the synthesized compounds.

Preferable substituted 2-amino-[1,2,4]triazolo[1,5-a]pyrimidine derivative represented by general formula (I) in a drug of the present invention are compounds shown in Table 6 below and 3-benzenesulfonyl-N-(7-thiophen-2-yl-[1,2,4]triazolo[1,5-a]pyrimidin-2-yl)propionamide, 3-benzenesulfonyl-N-(7-(4-methoxyphenyl)-[1,2,4]triazolo[1,5-a]pyrimidin-2-yl)propionamide, (S)-2-(tert-butoxycarbonyl)amino-N-(7-thiophen-2-yl-[1,2,4]triazolo[1,5-a]butylamide, (S)-2-amino-N-(7-thiophen-2-yl-[1,2,4]triazolo[1,5-a]pyrimidin-2-yl) butylamide or (S)-2-amino-3-methyl-N-(7-thiophen-2-yl-[1,2,4]triazolo[1,5-a]pyrimidin-2-yl)butylamide is particularly preferable.

The pharmaceutically acceptable salt of a [1,2,4]triazolo[1,5-a]pyrimidine derivative of the present invention is prepared by salification operation of a conventional method and, for example, alkali metal salts such as sodium, potassium salt, lithium salt; alkaline earth metal salts such as calcium salt, magnesium salt; other metal salts such as aluminum salt, iron salt, zinc salt; ammonium salt and so on; organic amine salts such as morpholine salt, ethylenediamine salt, guanidine salt, diethylamine salt, triethylamine salt, dicyclohexylamine salt, procaine salt, diethanolamine salt, piperazine salt, tetramethylammonium salt; hydrohalic acid salts such as hydrofluoric acid salt, hydrochloride, hydrobromic acid salt, hydroiodic acid salt; inorganic acid salts such as nitrate, perchlorate, sulfate, phosphate; organic sulfonic acid salts such as methanesulfonate, trifluoromethanesulfonate, ethanesulfonate, benzensuplhonate, p-toluenesulfonate; organic acid salts such as acetate, malate, succinate, citrate, tartrate, oxalate, maleate; or amino acid salts such as ornithinate, glutamate, aspartate. Preferably it is a hydrohalic acid salt or an organic salt.

Prodrugs which generate a drug of the present invention under physiological condition (physiological condition which, for example, is described in pp. 163-198 in "development of a drug", Vol. 7 "Molecular design", Hirokawa Shoten, 1990) in a living body, for example, by oxidation reaction, reductive reaction, hydrolysis reaction and so on with an enzyme or a gastric acid are included in the present invention. Examples of prodrug include an acylated compound, alkylated compound, phosphorylated compound of the active compound which it can be produced by a conventional organic reaction.

Pharmaceutically acceptable solvates such as hydrate of 2-amino[1,2,4]triazolo[1,5-a]pyrimidine derivatives are included in the present invention entirely.

An antigen presentation inhibitor which comprises as an active ingredient a substituted 2-amino-[1,2,4]triazolo[1,5-a]pyrimidine derivative represented by the above general formula (I) which is used as a drug of the present invention and has antigen presentation inhibitory activity as shown in the following test examples or pharmaceutically acceptable salt thereof is also included in the present invention.

Furthermore, an immunosuppresant which comprises as an active ingredient the substituted 2-amino-[1,2,4]triazolo[1,5-a]pyrimidine derivative or pharmaceutically acceptable salt thereof is also included in the present invention.

A lymphocyte proliferation inhibitor, cell differentiation/maturation inhibitor, immunotolerance inducer which comprises as an active ingredient the substituted 2-amino-[1,2,4]triazolo[1,5-a]pyrimidine derivative or pharmaceutically acceptable salt thereof is also included in the present invention, and further, a therapeutic agent or a prophylactic agent for transplant rejection reaction or graft versus host reaction disease, a therapeutic agent or a prophylactic agent for autoimmune diseases, a therapeutic agent or a prophylactic agent for allergic diseases, a therapeutic agent or a prophylactic agent for inflammatory diseases, an antineoplastic drug are included in the present invention.

The antigen presentation inhibitory substance of the present invention can be used for a therapeutic agent or a prophylactic agent for acute rejection reaction in transplantation, graft-versus-host disease, chronic rejection reaction, induction of immune tolerance, etc. The transplant may be any organ such as bone marrow, kidney, liver, heart and pancreas. As for the relations between donor and host, any case including xenograft transplantation, allograft transplantation, transplantation with blood incompatibility is possible. It can be used for the purpose of immune inhibition and long-term survival of the transplanted organ in organ transplantation such as bone marrow transplantation, peripheral blood stem cell transplantation and umbilical cord blood stem cell transplantation used for cancer therapy, treatment of autoimmune diseases, gene therapy and regeneration medicine.

The therapeutic agent or prophylactic agent for autoimmune diseases specifically includes a therapeutic agent or prophylactic agent for rheumatoid arthritis, multiple sclerosis, systemic lupus erythematosus, discoid lupus erythematosus, Sjogren's syndrome, Crohn's disease, colitis ulcerosa, idiopathic thrombocytopenia, aplastic anemia, autoimme hepatitis, insulin-dependent diabetes, myasthenia gravis, polymyositis, scleroderma, mixed connective tissue disease, ankylosing spondylitis or chronic thyroiditis.

The therapeutic agent or prophylactic agent for allergic diseases specifically includes a therapeutic agent or prophylactic agent for atopic dermatosis, pollinosis, contact hypersensitivity, asthma, psoriasis or anaphylaxis.

The therapeutic agent or prophylactic agent for inflammatory diseases specifically includes a therapeutic agent or prophylactic agent for Behcet's disease, polyarteritis, sarcoidosis, glomerulonephritis, nephrotic syndrome, intractable vasculitis or Wegener's syndrome.

The antineoplastic drug includes a therapeutic agent for malignant tumor such as lymphomas, leukemia, brain tumor, lung cancer, pancreas cancer, gastric cancer, large bowel cancer.

The drug of the present invention can be administered as it is or as a pharmaceutical composition mixed with a pharmaceutical acceptable carrier. The drug form of the pharmaceutical composition may be any type. These preparations may contain other ingredients valuable for the treatment. The ratio of the active ingredient of the present invention in the preparation is 1 to 100 %, preferably around 10 to 90%by weight for the whole preparation.

The drug of the present invention may be administered in any manner inclusing oral administration, injection, intrarectal administration, intraportal administration, visceral perfusion, local administration to an organ. The dosage of the drug of the present invention may vary dependent on administration method, applicable disease, pathological conditions age, body weight of a patient, but a usual dose is 0.01 mg to 500 mg/kg, preferably 0.05 mg to 50 mg/kg, which may be administered once a day or divided into several times a day. The administration can be performed one day alone or day after day, and repetitive administration may be performed with an interval of several days to several months. The administration method, dosage and administration schedule other than the above can be used as required.

The [1,2,4]triazolo[1,5-a]pyrimidine derivative represented by general formula (I) in the present invention can be obtained, for example, by the method shown in the following Formula C in the case where R is an alkyl group, can be obtained, for example, by the method shown in the following Formula A or B in the case where R is an alkoxycarbonyl group, an aliphatic acyl group, an amino acid group or a cyclic acyl group. In the following reaction formula, Ar represents the meaning same as the above. The compound in the reaction may form a salt which includes, for example, those similar to the salt of the [1,2,4]triazolo[1,5-a]pyrimidine derivatives represented by above general formula (I) (hereinafter compound (I)).

Formula A

[Formula 4]

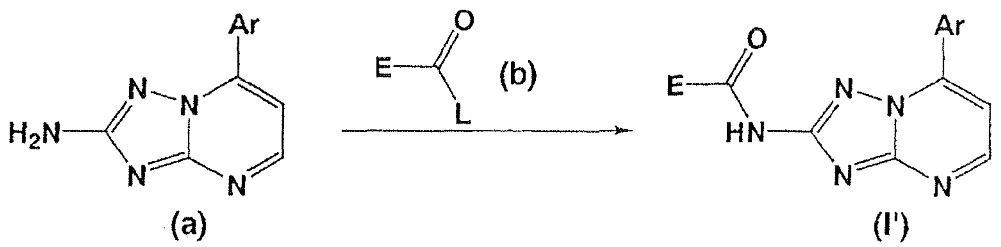

wherein E represents a residue of a (C2-C7) alkoxycarbonyl group in which ECO may be substituted with a substituent, (C1-C13) aliphatic acyl group which may have 1 to 3 same or different substituents, an amino acid group in which N-terminal may by protected or a 3- to 7-membered cyclic acyl group which may have 1 to 3 same or different substituents, and L represents a leaving group.)

The leaving group includes, for example, a halogeno group, a 4-nitrophenoxy group, a pentafluorophenoxy group, a

pyridin-2-ylsulfanyl group, an imidazol-1-yl group, a alkoxycarbonyl group, and preferably it is a halogeno group.

Compound (a) is reacted with compound (b) to obtain compound (I'). This reaction is usually performed in the absence of or in the presence of a base and preferably it is performed in the presence of a base. The base includes, for example, alkali metal carbonates such as lithium carbonate, sodium carbonate and potassium carbonate, alkaline metal bicarbonates such as lithium hydrogen carbonate, sodium hydrogen carbonate and potassium bicarbonate, alkali metal hydrides such as lithium hydride, sodium hydride and potassium hydride, alkali metal hydroxides such as lithium hydroxide, sodium hydroxide and potassium hydroxide, alkali metal alkoxides such as lithium methoxide, sodium methoxide, sodium ethoxide and potassium tert-butoxide, alkyl metals such as butyllithium and tert-butylmagnesium chloride, metal amides such as lithium diisopropylamide (LDA), lithium bis(trimethylsilyl)amide and sodium bis(trimethylsilyl)amide, organic amines such as triethylamine, tributylamine, diisopropylethylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diazabicyclo[2.2.2]octane (DABCO), 1,8-diazabicyclo[5.4.0]-7-undecene (DBU), and preferably it is an alkali metal hydride, a metal amide or an organic amine, and more preferably it is sodium hydride, lithium bis(trimethylsilyl)amide, diisopropylethylamine or pyridine. The amount of the base to be used is 0.5 to 5 mol equivalent, preferably 1 to 2 mol equivalent for compound (a). The amount of compound (b) to be used is 0.5 to 5 mol equivalent, preferably 1 to 2 mol equivalent for compound (a). A catalyst such as 4-(N, N-dimethylamino) pyridine in catalytic amount may be added if necessary.

This reaction is performed in the absence of or in the presence of a solvent and preferably it is performed in the presence of a solvent. The solvent is not particularly limited as far as the reaction proceeds and includes, for example, aliphatic hydrocarbons such as hexane, heptane, ligroin and petroleum ether, aromatic hydrocarbons such as benzene, toluene, xylene, halogenated hydrocarbons such as methylene chloride, chloroform and 1,2-dichloroethane, ethers such as diethyl ether, diisopropyl ether, tetrahydrofuran, dioxane, dimethoxyethane, diethylene glycol dimethyl ether, nitriles such as acetonitrile and propionitrile, amides such as dimethylformamide, dimethylacetamide and hexamethylphosphoric triamide, ureas such as 1,3-dimethylimidazolidin-2-one or a mixture of the above solvents, and preferably it is a halogenated hydrocarbon, an ether or a urea, and more preferably it is methylene chloride, tetrahydrofuran or 1,3-dimethylimidazolidin-2-one. The reaction temperature is usually -80 to 150°C, preferable 10 to 50°C. The reaction time is usually from 10 minutes to 48 hours.

When compound (a) or compound (b) has a reactive group such as a hydroxyl group or an amino group, this reaction is preferably performed while protecting with a protecting group which is used in a conventional reaction. When compound (b) is commercially available, the commercial article may be just used or the compound may be prepared according to a known method or a method following the same.

Particularly in the case where L of the above compound (b) is a fluorine atom, the compound can be derived from a compound in which L is a hydroxyl group. The fluorination reagent to be used includes, for example, cyanuric fluoride, TFFH (tetramethylfluoroformamidinium hexafluorophosphate), DAST ((dimethylamino) sulfur trifluoride) and preferably it is TFFH. The amount of the fluorination reagent to be used is 0.5 to 5 mol equivalent, preferably 1 to 2 mol equivalent for the compound in which L is a hydroxyl group. The fluorination reaction is performed in the absence of or in the presence of a base and preferably it is performed in the presence of a base. The base includes, for example, organic amines and the like such as triethylamine, tributylamine, diisopropylethylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diazabicyclo[2.2.2]octane (DABCO), 1,8-diazabicyclo[5.4.0]-7-undecene (DBU), and preferably it is diisopropylethylamine or pyridine. The amount of the base to be used is 0.5 to 5 mol equivalent, preferably 1 to 2 mol equivalent for the compound in which L is a hydroxyl group.

The fluorination reaction is performed in the absence of or in the presence of a solvent, and preferably it is performed in the presence of a solvent. The solvent is not particularly limited as far as the reaction proceeds and includes, for example, aliphatic hydrocarbons such as hexane, heptane, ligroin and petroleum ether, aromatic hydrocarbons such as benzene, toluene, xylene, halogenated hydrocarbons such as methylene chloride, chloroform and 1,2-dichloroethane, ethers such as diethyl ether, diisopropyl ether, tetrahydrofuran, dioxane, dimethoxyethane, diethylene glycol dimethyl ether, nitriles such as acetonitrile and propionitrile, amides such as dimethylformamide, dimethylacetamide and hexamethylphosphoric triamide, ureas such as 1,3-dimethylimidazolidin-2-one or a mixture of the above solvents, and preferably it is a halogenated hydrocarbon, an ether or a urea, and more preferably it is methylene chloride, tetrahydrofuran or 1,3-dimethylimidazolidin-2-one. The reaction temperature is usually -80 to 150°C, preferably 10 to 50°C. The reaction time is usually from 10 minutes to 12 hours. When the compound in which L is a hydroxyl group is commercially available, the commercial article may be just used or the compound may be prepared according to a known method or a method following the same. The compound obtained in the fluorination reaction may be isolated according to a conventional method, but may be used for the next reaction as a reaction liquid without isolating it.

When L of compound (b) is a fluorine atom, the reaction with the above compound (a) is performed particularly preferably in the presence of 1 to 2 equivalent amount of an alkali metal hydride or a metal amide in tetrahydrofuran or 1,3-dimethylimidazolidin-2-one at a reaction temperature of -10 to 50°C as a reaction condition.

In addition, when L of the above compound (b) is a chlorine atom, the compound can be derived from a

compound in which L is a hydroxyl group. The chlorination reagent to be used includes, for example, thionyl chloride, oxalyl chloride, cyanuric chloride, and preferably it is oxalyl chloride. The chloridization reaction is performed under the condition similar to that of the fluorination reaction mentioned above, and preferable reaction condition is the same.

Formula B

[Formula 5]

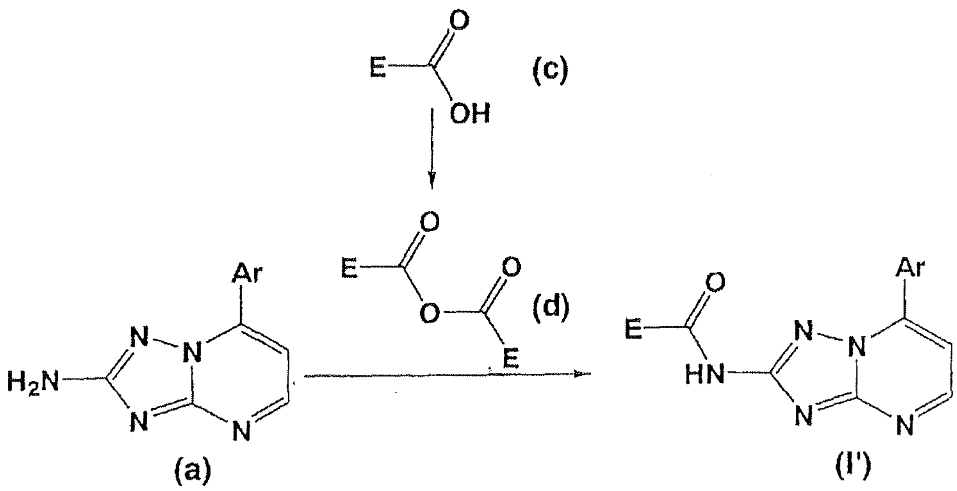

wherein E represents the same meaning as E in the above Formula A).

A production process in which compound (c) is converted into acid anhydride (d) by a dehydration condensation agent and it is reacted with compound (a), and thereby obtaining a substituted 2-amino-[1,2,4]triazolo[1,5-a]pyrimidine derivative (I') is described. Usable dehydration condensation agent includes, for example, dicyclohexylcarbodiimide, diisopropylcarbodiimide, 1-dimethylaminopropyl-3-ethylcarbodiimide, and preferably it is diisopropylcarbodiimide. The amount of the dehydration condensation agent to be used is 0.3 to 0.8 mol equivalent, preferably 0.5 mol equivalent for compound (c).

The dehydration condensation reaction is performed in the absence of or in the presence of a solvent, and preferably it is performed in the presence of a solvent. The solvent is not particularly limited as far as the reaction proceeds and includes, for example, aliphatic hydrocarbons such as hexane, heptane, ligroin and petroleum ether, aromatic hydrocarbons such as benzene, toluene, xylene, halogenated hydrocarbons such as methylene chloride, chloroform and 1,2-dichloroethane, ethers such as diethyl ether, diisopropyl ether, tetrahydrofuran, dioxane, dimethoxyethane, diethylene glycol dimethyl ether, nitriles such as acetonitrile and propionitrile, ureas such as 1,3-dimethylimidazolidin-2-one or a mixture of the above solvents, and preferably it is a halogenated hydrocarbon, an ether or a urea, and more preferably it is methylene chloride, tetrahydrofuran or 1,3-dimethylimidazolidin-2-one. The reaction temperature is usually -20 to 100°C, preferably -10 to 50°C. The reaction time is usually from several minutes to 12 hours. When compound (c) is commercially available, the commercial article may be just used or the compound may be prepared according to a known method or a method following the same. The compound obtained in the dehydration condensation reaction may be isolated according to a conventional method, but may be used for the next reaction as a reaction solution without isolating it.

The amount of acid anhydride (d) to be used in the reaction of compound (a) with the acid anhydride (d) obtained by the above method is 0.5 to 4 mol equivalent, preferably 1.0 to 3.0 mol equivalent for compound (a). This reaction is performed in the absence of or in the presence of a solvent, and preferably it is performed in the presence of a solvent. As for the solvent, those solvents shown in the above Formula A can be used. The reaction temperature is usually from room temperature to 150°C and preferably 80 to 120°C. The reaction time is usually 1 to 12 hours. When compound (a) or compound (c) has a reactive group such as a hydroxyl group or an amino group, this reaction is preferably performed while protecting with a protecting group.

Formula C

[Formula 6]

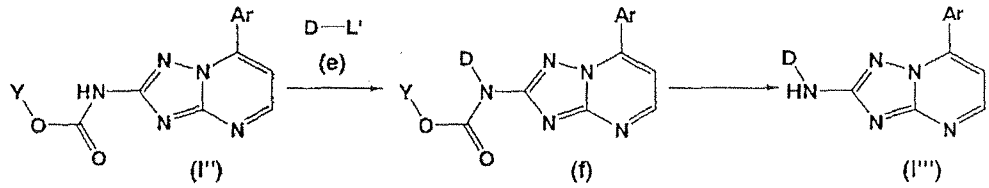

(I'') (f) (I''')

wherein D represents a (C1-C6) alkyl group which may have a substituent, L' represents a leaving group, and Y represents a (C1-C6) alkyl group which may have a substituent.
The leaving group includes, for example, a chloro group, a bromo group, a iodo group, a methane sulfonyloxy group, a p-toluenesulfonyl group.

After compound (e) is reacted with compound (I'') obtained by Formula A or also by Formula B and so on to obtain compound (f), compound (I''') is obtained by dealkoxycarbonylation reaction. The first step reaction is usually performed in the presence of a base, and the base includes, for example, alkali metal carbonates such as lithium carbonate, sodium carbonate and potassium carbonate, alkaline metal bicarbonates such as lithium hydrogen carbonate, sodium hydrogen carbonate and potassium bicarbonate, alkali metal hydrides such as lithium hydride, sodium hydride and potassium hydride, alkali metal hydroxides such as lithium hydroxide, sodium hydroxide and potassium hydroxide, alkali metal alkoxides such as lithium methoxide, sodium methoxide, sodium ethoxide and potassium tert-butoxide, alkyl metals such as butyllithium and tert-butylmagnesium chloride, metal amides such as lithium diisopropylamide (LDA), lithium bis(trimethylsilyl)amide and sodium bis(trimethylsilyl)amide, organic amines such as triethylamine, tributylamine, diisopropylethylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diazabicyclo[2.2.2]octane (DABCO), 1,8-diazabicyclo[5.4.0]-7-undecene (DBU), and preferably it is an alkali metal hydride, a metal amide or an organic amine, and more preferably it is sodium hydride, lithium bis(trimethylsilyl)amide, diisopropylethylamine or pyridine. The amount of the base to be used is 0.5 to 5 mol equivalent, preferably 1 to 2 mol equivalent for compound (I''). The amount of compound (e) to be used is 0.5 to 5 mol equivalent, preferably 1 to 2 mol equivalent for compound (I'').

This reaction is performed in the absence of or in the presence of a solvent, and preferably it is performed in the presence of a solvent. The solvent is not particularly limited as far as the reaction proceeds and includes, for example, aliphatic hydrocarbons such as hexane, heptane, ligroin and petroleum ether, aromatic hydrocarbons such as benzene, toluene, xylene, halogenated hydrocarbons such as methylene chloride, chloroform and 1,2-dichloroethane, ethers such as diethyl ether, diisopropyl ether, tetrahydrofuran, dioxane, dimethoxyethane, diethylene glycol dimethyl ether, nitriles such as acetonitrile and propionitrile, amides such as dimethylformamide, dimethylacetamide and hexamethylphosphoric triamide, ureas such as 1,3-dimethylimidazolidin-2-one or a mixture of the above solvents, and preferably it is an ether, an amide or a urea, and more preferably it is tetrahydrofuran, dimethylformamide or 1,3-dimethylimidazolidin-2-one. The reaction temperature is usually -80 to 150°C, preferably 0 to 80°C. The reaction time is usually from 10 minutes to 48 hours. When compound (I'') has a reactive group such as a hydroxyl group or an amino group, this reaction is preferably performed while protecting with a protecting group. When compound (e) is commercially available, the commercial article may be just used or the compound may be prepared according to a known method or a method following the same.

The second step reaction is performed under a condition of conventional de-alkoxycarbonylation reaction. Y in this reaction preferably includes, for example, a tert-butyl group or a benzyl group, and when it is tert-butyl group, the reaction is performed with an acid such as trifluoroacetic acid and hydrochloric acid and when x is a benzyl group, the reaction is performed by reduction reaction under hydrogen atmosphere with palladium carbon and the like.

Compound (a) used in the above Formula A or B can be obtained by a process described in the following Formula D which follows a known method for producing a [1,2,4]triazolo[1,5-a]pyrimidine derivative, for example, those described in JP-A-04-099775, US Patent No.4,444,774, Reiter, J. et al., Tetrahedron, vol. 43, 2497-2504 (1987).

Formula D

[Formula 7]

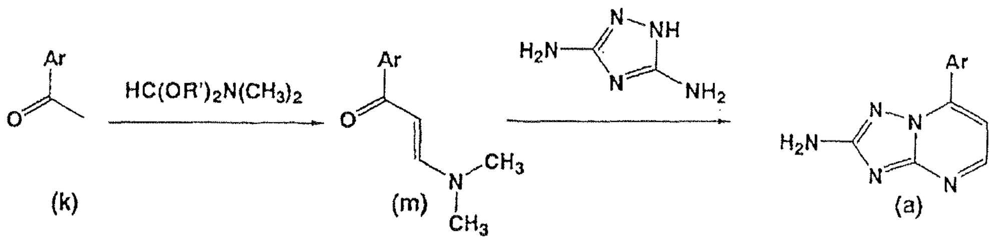


wherein Ar represents the meaning same as the above, and R represents a (C1-C6) alkyl group.)

Acetophenones (k) and a formamide dimethylacetal compound are condensed to obtain compound (m). The amount of formamide dimethylacetal compound to be used is 0.5 to 5 mol equivalent, preferably 1 to 3 mol equivalent for compound (k). This reaction is performed in the absence of or in the presence of a solvent. The solvent is not particularly limited as far as the reaction proceeds and includes, for example, aliphatic hydrocarbons such as hexane, heptane, ligroin and petroleum ether, aromatic hydrocarbons such as benzene, toluene, xylene, halogenated hydrocarbons such as methylene chloride, chloroform and 1,2-dichloroethane, ethers such as diethyl ether, diisopropyl ether, tetrahydrofuran, dioxane, dimethoxyethane, diethylene glycol dimethyl ether, or a mixture of the above solvents, and preferably it is an aromatic hydrocarbons such as benzene, toluene, xylene. The reaction temperature is usually 80 to 200°C, preferably 110 to 150°C. The reaction time is usually from 6 to 48 hours. As for compound (k) and a formamide dimethylacetal compound, when they are commercially available, the commercial articles may be just used or the compounds may be prepared according to a known method or a method following the same.

Then, compound (m) and 3,5-diamino-1,2,4-triazole are condensed to obtain compound (a). This reaction is performed in the absence of or in the presence of an acid, and preferably it is performed in the presence of an acid. The acid includes, for example, mineral acids such as hydrochloric acid and sulfuric acid, carboxylic acids such as acetic acid, benzoic acid and trifluoroacetic acid, sulfonic acids such as methanesulfonic acid, trifluoromethane sulfonic acid, p-toluenesulfonic acid and camphor sulfonic acid, Lewis acids such as boron trifluoride, titanium tetrachloride and stannic tetrachloride, and preferably it is a sulfonic acid. The amount of the acid to be used is 0.1 mol equivalent to large excess, preferably 0.2 to 2 mol equivalent for compound (m). The amount of 3,5-diamino-1,2,4-triazole to be used is 0.5 to 10 mol equivalent, preferably 1 to 4 mol equivalent for compound (m).

This reaction is performed in the absence of or in the presence of a solvent, and the solvent is not particularly limited as far as the reaction proceeds and includes, for example, aliphatic hydrocarbons such as hexane, heptane, ligroin and petroleum ether, aromatic hydrocarbons such as benzene, toluene, xylene, halogenated hydrocarbons such as methylene chloride, chloroform and 1,2-dichloroethane, ethers such as diethyl ether, diisopropyl ether, tetrahydrofuran, dioxane, dimethoxyethane, diethylene glycol dimethyl ether, nitriles such as acetonitrile and propionitrile, amides such as dimethylformamide, dimethylacetamide and hexamethylphosphoric triamide, ureas such as 1,3-dimethylimidazolidin-2-one, carboxylic acids such as acetic acid and propionic acid or a mixture of the above solvents, and preferably it is an aromatic hydrocarbon. The reaction temperature is usually 50 to 150°C, preferably 80 to 120°C. The reaction time is usually from 10 minutes to 6 hours.

Furthermore, substituents on Ar and R in a compound represented by general formula (I) can be produced by converting according to a conventional method or a method following the same, as required, for example, by reaction such as esterification reaction, amidation reaction, hydrolysis reaction, oxidation reaction, reductive reaction, alkylation reaction, conventional deprotection reaction or the like.

When a product is obtained in a free form, it can be converted to its salt, and when a product is obtained as a salt, it can be converted to its free form according to a conventional method, respectively in each of the above processes.

As for a protecting group when a reactive group such as an amino group and a hydroxyl group is included in a substituent in the above reaction, known methods (for example, a method described in Greene, T.W. et al., "PROTECTIVE GROUPS IN ORGANIC SYNTHESIS", second edition, WILEY INTERSCIENCE (U.S.A.)) can be used.

The protecting group of an amino group includes, for example, a (C1-C7) acyl group, a benzoyl group, a (C2-C7) alkoxycarbonyl group, a benzyloxycarbonyl group, a phthaloyl group.

The protecting group of a hydroxyl group includes, for example, a (C1-C6) alkyl group, a benzyl group, a (C1-C7) acyl group, a benzoyl group, an alkylsubstituted silyl group.

Each product in each of the above processes can be isolated and purified according to known separation means, for example, by distillation, vacuum concentration, solvent extraction, crystallization and chromatography.

The present invention also includes a substituted 2-amino[1,2,4]triazolo[1,5-a]pyrimidine derivative represented

by the above-mentioned general formula (III) wherein A represents a phenyl group which may be substituted with 1 to 3 same or different substituents selected from the substituent group (A) consisting of a halogeno group, a hydroxyl group, a (C1-C6) alkyl group, a cyano group, a nitro group, a (C1-C6) alkoxyl group, a benzyloxy group, an amino group, a (C1-C7) acylamino group and a methylenedioxy group or a 5- or 6-membered aromatic heterocyclic group which contains one hetero atom and may be substituted with 1 to 3 same or different substituents selected from the substituent group (A);
W represents a (C1-C6) alkyl group which may be substituted with one substituent selected from the substituent group consisting of a halogeno group, a hydroxyl group, a (C1-C6) alkoxyl group, a phenyl group and a 5- or 6-membered aromatic heterocyclic group which contains one hetero atom;
a (C2-C7) alkoxycarbonyl group which may be substituted with one substituent selected from the substituent group consisting of a phenyl group, a methoxyphenyl group and a 5- or 6-membered aromatic heterocyclic group which contains one hetero atom;
a (C1-C13) aliphatic acyl group which may have 1 to 3 same or different substituents selected from the substituent group consisting of a halogeno group, a hydroxyl group, an oxo group, a (C1-C6) alkoxyl group, a (C1-C7) acyl group, a (C1-C7) acyloxy group, a trifluoromethyl group, a trifluoromethoxy group, a cyano group, a nitro group, a (C1-C6) alkylsulfanyl group, a benzylsulfanyl group, an arylsulfanyl group, a (C1-C6) alkylsulfonyl group, an arylsulfonyl group, a (C1-C6) alkoxycarbonyl group, an amino group, a (C1-C6) alkylamino group, a di(C1-C6) alkylamino group, a (C1-C7) acylamino group, a (C1-C6) alkoxycarbonylamino group, a benzyloxycarbonylamino group, a phenyl group, wherein the phenyl group may be substituted with 1 to 3 substituents selected from the substituent group consisting of a halogeno group, a (C1-C6) alkyl group, a trifluoromethyl group, a cyano group, a nitro group, a (C1-C6) alkoxyl group, a benzyloxy group, an amino group, a di(C1-C6) alkylamino group, or a 5- or 6-membered saturated or unsaturated heterocyclic group which contains 1 to 4 hetero atoms independently selected from N, O and S and may be substituted with 1 to 3 (C1-C6) alkyl groups;
an α-amino acid group which may be protected at the N-terminal; or
a cyclic acyl group represented by Z-CO-,
wherein Z represents an aromatic hydrocarbon group which may have 1 to 3 same or different substituents selected from the substituent group (B) consisting of a (C1-C6) alkyl group, a halogeno group, a hydroxyl group, an oxo group, a trifluoromethyl group, a cyano group, a nitro group, a (C1-C6) alkoxyl group, a benzyloxy group, a (C1-C6) alkylsulfanyl group, a (C1-C6) alkoxycarbonyl group, an amino group, a di(C1-C6) alkylamino group, a (C1-C7) acylamino group and a methylenedioxy group, or a 5- or 6-membered saturated or unsaturated heterocyclic group which contains 1 to 4 hetero atoms independently selected from N, O and S and may be substituted with 1 to 3 same or different substituents selected from the substituent group (B), or a pharmaceutically acceptable salt thereof.

Preferable compounds as a substituted 2-amino-[1,2,4]triazolo[1,5-a]pyrimidine derivative of the present invention include 3-benzenesulfonyl-N-(7-thiophen-2-yl-[1,2,4]triazolo[1,5-a]pyrimidin-2-yl)propionamide, 3-benzenesulfonyl-N-(7-(4-methoxyphenyl)-[1,2,4]triazolo[1,5-a]pyrimidin-2-yl)propionamide, (S)-2-(tert-butoxycarbonyl)amino-N-(7-thiophen-2-yl-[1,2,4]triazolo[1,5-a]pyrimidin-2-yl)butylamide, (S)-2-amino-N-(7-thiophen-2-yl-[1,2,4]triazolo[1,5-a]pyrimidin-2-yl)butylamide or (S)-2-amino-3-methyl-N-(7-thiophen-2-yl-[1,2,4]triazolo[1,5-a]pyrimidin-2-yl)butylamide or pharmaceutically acceptable salts thereof.

The present invention is further described by way of the following test examples.

Test Example 1: Effect on MHC class I expression of T1 cell (Human lymphoma line 174 × CEM.)
T1 cells ($5.7 \times 10^3$ cells/200 μL/well) were incubated using a 96-well flat bottom microplate in RPMI1640 culture medium (Iwaki Glass Corporation) containing 10% bovine serum (hereinafter referred to as FBS; Iruvine Scientific) in the presence of a test compound of each concentration diluted from 400 μM by a common ratio of root 10 at 37°C in an incubator containing 5% carbon dioxide concentration for three days. After incubation, cells were stained with fluoroisothiocyanate labeled mouse antihuman MHC class I monoclonal antibody (antibody produced by cell strain W6/32 (ATCC No, CRL1991). Mean fluorescence intensity (hereinafter referred to as MFI) of stained cells was measured with flow cytometry FACScan (BD), and this was assumed as MHC class I molecule expression level. Concentrations of the test compounds which inhibited the expression level of MHC class I molecule by 20% ($EC_{20}$ value) were calculated from the following Expression (1), which are shown in Table 1.

[Expression 1]

$$\text{Suppression ratio of MHC class I expression (\%)} = \left(1 - \frac{E\ exp}{C\ exp}\right) x100$$

Eexp: MFI of antibody stained cells cultured with addition of a test compound
Cexp: MFI of antibody stained cells cultured without addition of a test compound
[Table 1]

| Test Compound Example Number | $EC_{20}$ (μM) | Test Compound Example Number | $EC_{20}$ (μM) |
|---|---|---|---|
| 001 | 6.0 | 026 | 8.8 |
| 007 | 6.25 | 028 | 7.7 |
| 008 | 7.5 | 036 | 7.1 |
| 009 | 7.2 | 040 | 8.4 |
| 013 | 8.2 | 047 | 7.3 |
| 015 | 9.2 | 052 | 6.8 |
| 016 | 9.7 | 054 | 4.0 |
| 020 | 5.3 | 055 | 1.6 |
| 022 | 8.2 | 056 | 0.74 |

It was shown from these results that the compounds of the present invention inhibited MHC class I expression of T1 cells.
Test Example 2: Effect on MHC class I expression of dendritic cell derived from human peripheral blood
Mononuclear leukocytes were separated from human peripheral blood by specific gravity centrifugal method and CD14 positive cells were separated the peripheral blood mononuclear leukocytes using mouse antihuman CD14 antibody binding microbeads and a magnetic cell separation system (Miltenyi Biotec GmbH). The separated CD14 positive cells were floated in RPMI1640 culture medium containing 10% FBS, and disseminated on a 6-well plate to attain $1.0 \times 10^6$ cells/ well and incubated at 37°C in an incubator containing 5% carbon dioxide concentration for 20 minutes. After incubation, non-adherent cells which did not have adhesive property and floated in the solution were removed. To this were added 500 U/mL of human recombinant granulocyte colony-stimulating factor (hereinafter referred to as GM-CSF; Anapure Bioscientific), 50 ng/mL of human recombinant interleukin 4 (hereinafter referred to as IL-4; PeproTech) and 2 mL/well of RPMI1640 containing 10% FBS and incubated at 37°C in an incubator containing 5% carbon dioxide concentration. When CD14 positive cells with adhesive property were cultured in the presence of GM1:SF, IL-4, the CD14 positive cells differentiates to immature dendritic cells with non-adhesive property. After having been incubated for 7 days, immature dendritic cells were obtained by collecting non-adhesive cells. The immature dendritic cells ($2 \times 10^5$ cells/well) were incubated using a 6-well plate in RPMI1640 culture medium containing 10% FBS to which 100 U/mL of human recombinant tumor necrosis factor-α (hereinafter referred to as TNF-α; PeproTech) and a test compound were added at 37°C in an incubator containing 5% carbon dioxide concentration for three days. The dendritic cells matured by TNF-α but a test compound was acted on them in this test at the same time. These matured dendritic cells were stained with fluoroisothiocyanate labeled mouse antihuman MHC class I monoclonal antibody (antibody produced by cell strain W6/32 (ATCC No, CRL1991) and the ratio of number of MHC class I molecule highly expressing cells/all cells was measured with flow cytometry. Decrement of MHC class I molecule highly expressing cells was calculated from the following Expression (2) and the concentrations of the test compounds which showed 50% decrement ($EC_{50}$ value) were determined, which are shown in Table 2.
[Expression 2]

$$\text{Reduction rate of number of MHC class I molecule highly expressing cells (\%)} = \left(1 - \frac{E\,exp}{C\,exp}\right) x100$$

Eexp: (Number of MHC class I molecule highly expressing cells cultured with addition of a test compound)/(All number)
Cexp: (Number of MHC class I molecule highly expressing cells cultured without addition of a test compound)/(All number)
[Table 2]

| Test Compound Example Number | $EC_{20}$ ($\mu$M) |
|---|---|
| 055 | 6.7 |
| 056 | 6.3 |

It was shown that the compounds of the present invention inhibited MHC class I expression of dendritic cells derived from human peripheral blood.

Test Example 3: Effect on ability of human dendritic cell inducing proliferation of allogeneic T cell

Immature dendritic cells were incubated with a test compound and TNF-$\alpha$ for three days in the same manner as in the method described in Test Example 2. The obtained dendritic cells ($2.5 \times 10^3$ cells/50 $\mu$L/well) were incubated using a 96-well flat bottom plate in a mixed condition with human allogeneic T cells ($2.0 \times 10^6$ cells/150 $\mu$L/well) at 37°C in an incubator containing 5% carbon dioxide concentration for five days. 1 $\mu$Ci/10 $\mu$L/ well of [$^3$H]-thymidine (Amersham Pharmacia Biotech) was added 16 hours before culture termination. After the culturing was terminated, cells were collected using cell harvester (Skatron Instrument) on a glass filter and after they were dried, scintillator ACS-II (Amersham Pharmacia Biotech) was added, and the radioactivity by [$^3$H]-thymidine taken up into the cells was measured using a liquid scintillation counter. DNA synthesis inhibition ratio of lymphocyte was calculated from the following Expression (3) and the concentrations of the test compounds which showed 50% inhibition ($IC_{50}$ value) were determined, which are shown in Table 3.

[Expression 3]

$$\text{DNA synthesis inhibition ratio of lymphocyte (\%)} = \left(1 - \frac{\text{E exp}}{\text{C exp}}\right) \times 100$$

Eexp: Uptake amount of [$^3$H]-thymidine by cells cultured with addition of a test compound

Cexp: Uptake amount of [$^3$H]-thymidine by cells cultured without addition of a test compound

[Table 3]

| Test Compound Example Number | $EC_{20}$ ($\mu$M) |
|---|---|
| 028 | 24.1 |
| 044 | 27.5 |

It was shown that the compounds of the present invention inhibited the ability of dendritic cell to induce proliferation of lymphocyte.

Test Example 4: Cytotoxic effect on T1 cell (Human lymphoma line 174 $\times$ CEM.)

T1 cells ($5.7 \times 10^3$ cells/200 $\mu$L/well) were incubated using a 96-well flat bottom microplate in RPMI1640 culture medium containing 1.0% FBS in the presence of a test compound of each concentration diluted from 400 $\mu$M by a common ratio of root 10 at 37°C in an incubator containing 5% carbon dioxide concentration for three days. After incubation, damaged cells were stained with propodium iodide. $IC_{50}$ values were determined from the stained number to all number measured with flow cytometry FACScan (BD), and this was assumed as cytotoxic activity. The results are shown in Table 4.

[Table 4]

| Test Compound Example Number | $EC_{20}$ (μM) |
|---|---|
| 029 | 1.3 |
| 030 | 14.1 |
| 051 | 3.2 |

It was shown that the compounds of the present invention have ytotoxic effect on T1 cells and anti cell proliferation inhibition activity.

Hereinafter, the present invention is described more in detail with reference to Examples, Reference Examples and Test Examples, but the present invention is not limited to these.

Reference Example 001: Synthesis of 7-thiophene-triazolo[1,2,4][1,5-a]pyrimidin-2-ylamine
2-acetylthiophene (8.20 g) and N,N-dimethylformamide diethyl acetal (13.6 mL) were heated to reflux at 130°C in xylene (13 mL) for two days. The reaction liquid was concentrated under reduced pressure, and after subjected to azeotropic distillation with toluene, crystallized with a mixed solvent of toluene-hexane and 3-dimethylamino-1-thiophen-2-ylpropenone (11.52g, yield 98%) was obtained.
The obtained 3-dimethylamino-1-thiophen-2-ylpropenone (10.78 g) was dissolve in toluene (160 mL) and stirred at 100°C with 3,5-diamino-1,2,4-triazole (14.15 g) added. After 10-camphor sulfonic acid (13.82 g) was added, it was heated to reflux for 1.5 hours. After having cooled to room temperature, the supernatant was removed (decantation). The residue was suspended and washed with 5% sodium carbonate/10% ethanol aqueous solution, 10% ethanol water, absolute ethanol and methylene chloride in this order and dried under reduced pressure and the title compound was obtained (8.55 g, yield 66%).

Reference Example 002: Synthesis of 7-(4-nitrophenyl)-[1,2,4]triazolo[1,5-a]pyrimidin-2-ylamine
4'-nitroacetophenone was used in place of 2-acetylthiophene in Reference Example 001 and heated to reflux in xylene in the presence of N,N-dimethylformamide diethyl acetal as in Reference Example 001, and 3-dimethylamino-1-(4-nitrophenyl)propenone (quantitative) was obtained, which was subsequently reacted with 3,5-diamino-1,2,4-triazole in xylene in the presence of 10-camphor sulfonic acid and the title compound was obtained (yield 82%).

Reference Example 003: Synthesis of 7-(4-nitrophenyl)-[1,2,4]triazolo[1,5-a]pyrimidin-2-ylamine
7-(4-aminophenyl)[1,2,4]triazolo[1,5-a]pyrimidin-2-ylamine (62.0 mg) obtained in Reference Example 002 was suspended in acetic acid (0.8 mL) and stirred at room temperature overnight with tin (II) chloride dihydrate (200 mg) and concentrated hydrochloric acid (0.6 mL) added. The reaction liquid was neutralized with 6M aqueous sodium hydroxide solution and the sedimented deposit was separated by filtration. It was suspended and washed with distilled water, ethanol and methylene chloride in this order and the title compound was obtained (42.3 mg, yield 77%).

Reference Example 004: Synthesis of 7-(4-aminophenyl)[1,2,4]triazolo[1,5-a]pyrimidin-2-ylamine
7-(4-nitrophenyl)-[1,2,4]triazolo[1,5-a]pyrimidin-2-ylamine (20.1 mg)obtained in Reference Example 003 was suspended in methanol (2 mL) and stirred for all one day with acetic acid anhydride (16. 9 μL) added. After 5% potassium carbonate aqueous solution (5 mL) was added to the reaction liquid, which was subjected to centrifugal separation (2000rpm, 10 minutes). The supernatant was removed and the precipitation was suspended and washed with ethanol and methylene chloride in this order and the title compound was obtained (19.2 mg, yield 81%).

Reference Example 005: Synthesis of 7-(4-methoxyphenyl)-[1,2,4]triazolo[1,5-a]pyrimidylamine
4'-methoxy acetophenone was used in place of 2-acetylthiophene in Reference Example 001 and heated to reflux in xylene in the presence of N,N-dimethylformamide diethyl acetal as in Reference Example 001, and 3-dimethylamino-1-(4-methoxyphenyl)propenone (yield 99%) was obtained, which was subsequently reacted with 3,5-diamino-1,2,4-triazole in toluene in the presence of 10-camphor sulfonic acid and the title compound was obtained (yield 61%).

Reference Example 006: Synthesis of 7-(3-chlorophenyl)-[1,2,4]triazolo[1,5-a]pyrimidin-2-ylamine
3'-chloroacetophenone was used in place of 2-acetylthiophene in Reference Example 001 and heated to reflux in xylene in the presence of N,N-dimethylformamide diethyl acetal as in Reference Example 001, and 3-dimethylamino-1-(3-chlorophenyl)propenone (quantitative) was obtained, which was subsequently reacted with 3,5-diamino-1,2,4-triazole in toluene in the presence of 10-camphor sulfonic acid and the title compound was obtained (yield 44%).

Reference Example 007: Synthesis of 7-phenyl[1,2,4]triazolo[1,5-a]pyrimidin-2-ylamine
Acetophenone was used in place of 2-acetylthiophene in Reference Example 001 and heated to reflux in xylene in the presence of N,N-dimethylformamide diethyl acetal as in Reference Example 001, and 3-dimethylamino-1-phenylpropenone (yield 41%) was obtained, which was subsequently reacted with 3,5-diamino-1,2,4-triazole in toluene in the presence of 10-camphor sulfonic acid and the title compound was obtained (yield 63%).

Reference Example 008: Synthesis of 7-(3-methoxyphenyl)-[1,2,4]triazolo[1,5-a]pyrimidin-2-ylamine

3'-methoxyacetophenone was used in place of 2-acetylthiophene in Reference Example 001 and heated to reflux in xylene in the presence of N,N-dimethylformamide diethyl acetal as in Reference Example 001, and 3-dimethylamino-1-(3-methoxyphenyl)propenone (quantitative) was obtained, which was subsequently reacted with 3,5-diamino-1,2,4-triazole in toluene in the presence of 10-camphor sulfonic acid and the title compound was obtained (yield 65%).
The structural formulas and physicochemical data of the compounds of Reference Examples 001-008 are shown in Table 5 below.

[Table 5]

| Reference Exam. No. | Structural Formula | Physicochemical Data |
|---|---|---|
| Reference Example 001 | 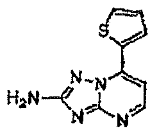 | MS: *m*/*z* 218 $(M+H)^+$. |
| Reference Example 002 | 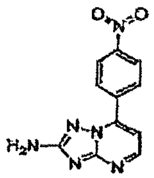 | $^1$H-NMR (DMSO-$d_6$, ppm): 6.57 (2H, brs), 7.36 (1H, d, J = 4.9 Hz), 8.30-8.60 (4H, overlapped), 8.61 (1H, d, J = 4.9 Hz). |
| Reference Example 003 | 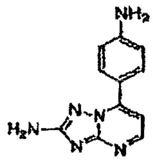 | MS: *m*/*Z* 227 $(M+H)^+$. |
| Reference Example 004 | 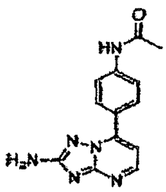 | MS: *m*/*Z* 269 $(M+H)^+$. |
| Reference Example 005 | 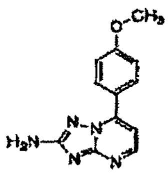 | $^1$H-NMR (DMSO-$d_6$, ppm): 3.87 (3H, s), 6. 45 (2H, brs), 7.10-7.22 (2H, m), 7.26 (1H, d, J = 5.1 Hz), 8.20-8.32 (2H, m), 8.49 (1H, d, J = 4.6 Hz);<br>MS: *m*/*Z* 242 $(M+H)^+$. |
| Reference Example 006 | 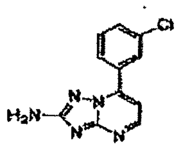 | MS: *m*/*Z* 246 $(M+H)^+$. |
| Reference Example 007 | 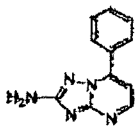 | MS: *m*/*Z* 212 $(M+H)^+$. |

Table continued

| Reference Exam. No. | Structural Formula | Physicochemical Data |
|---|---|---|
| Reference Example 008 | 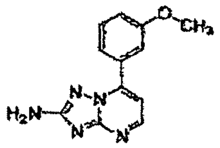 | $^1$H-NMR (DMSO-$d_6$, ppm): 3.85 (3H, s), 6.48 (2H, brs), 7.19 (1H, m), 7.29 (1H, d, J = 5.0 Hz), 7.52 (1H, dd, J = 7.7, 8.4 Hz), 7.68-7.80 (2H, overlapped), 8.54 (1H, d, J = 4.8 Hz); MS: *m/Z* 242 $(M+H)^+$. |

Example 001: Synthesis of N-methyl-7-thiophene-[1,2,4]triazolo[1,5-a]pyrimidin-2-ylamine
The compound of Example 005 (69.4 mg, 0.22 mmol) was dissolved in N,N-dimethylformamide (1.6 mL) and stirred at room temperature for one hour with sodium hydride (60% paraffin solution, 17.6 mg, 0.44mmol) and methyl iodide (27.4 μL, 0.44 mmol) added. After diluted with methylene chloride (5 mL), the mixture was washed with 0.4M hydrochloric acid water and distilled water. After the organic layer was concentrated under reduced pressure, hexane was added to the residue, which was suspended and washed to obtain pale brown powder (59.0 mg). Subsequently anisole (40 μL) and trifluoroacetic acid (1 mL) were added and stirred at room temperature for one hour, and the reaction liquid was concentrated under reduced pressure and the obtained residue was suspended and washed with hexane, 5% potassium carbonate aqueous solution and distilled water in this order, dried under reduced pressure and the title compound was obtained (31.0mg, yield 61%).
$^1$H-NMR(DMSO-$d_6$, ppm): 2.94(3 H, brd, J = 4.0 Hz), 7.04(1H, brq, J = 4.0 Hz), 7.4(1H, dd, J = 3.9, 5.0 Hz), 7.65(1H, d, J = 5.3Hz), 8.14(1H, dd, J = 1.1, 5.0Hz), 8.49(1H, d, J = 5.3 Hz) 8.51(1H, dd, J = 1.1, 3.9Hz)

Example 005: Synthesis of 7-thiophene[1,2,4]triazolo[1,5-a]pyrimidin-2-ylcarbamic acid tert-butyl ester (example of Reaction Formula A)
The compound of Reference Example 001 (234 mg, 1.08 mmol) was suspended in tetrahydrofuran (12 mL) and stirred at room temperature for 10 minutes with di-tert-butyldicarbonate (306 mg, 1.40 mmol) and hexamethyldisilazane lithium salt (1M tetrahydrofuran solution, 2 mL) added. After diluted with methylene chloride (40 mL), the mixture was washed with 1M hydrochloric acid water, distilled water, 5% potassium carbonate aqueous solution and saturated saline in this order. After the organic layer was dried with anhydrous sodium sulfate, it was concentrated under reduced pressure. The residue was crystallized from a mixed solvent of isopropyl ether/methylene chloride and the title compound was obtained (340mg, yield 99%).
$^1$H-NMR(DMSO-$d_6$, ppm): 1.52(9H, s), 7.43(1H, dd, J = 3.9, 5.0 Hz), 7.83(1H, d, J = 5.1 Hz), 8.20(1H, dd, J = 1.2, 5.0 Hz), 8.63(1H, dd, J = 1.2, 3.9 Hz), 8.72 (1H, d, J = 5.1 Hz), 10.51(1H, s)

Example 022: Synthesis of 3-benzenesulfonyl-N-(7-thiophene[1,2,4]triazolo[1,5-a]pyrimidin-2-yl)propionamide (example of Reaction Formula A)
3-(phenylsulfonyl)propionic acid (26.3 mg, 0.12 mmol) was dissolved in tetrahydrofuran (1 mL) and stirred at room temperature for one hour with oxalyl chloride (10.5 μL, 0.12 mmol) added. The compound of Reference Example 001 (21.7 mg, 0.10 mmol), pyridine (24.3 μL, 0.30 mmol) and 4-dimethylaminopyridine (2.4 mg, 0.02 mmol) were added to this reaction liquid and stirred at room temperature overnight. Distilled water (1 mL) and 2M aqueous sodium hydroxide solution (0. 25 mL) were added to the reaction liquid and extracted with methylene chloride (4 mL). After washed with 4M hydrochloric acid (3 mL) × 2, distilled water (3 mL), 5% potassium carbonate aqueous solution (3 mL) and then with distilled water (3 mL), the organic layer was concentrated under reduced pressure, and the title compound was obtained (14.6 mg, yield 35%).

Example 028: Synthesis of 6,6-dimethyl-4-oxo-5,6-dihydro-4H-pyran-2-carboxylic acid N-(7-thiophen-2-yl-[1,2,4]triazolo[1,5-a]pyrimidin-2-yl)amide (example of Reaction Formula A)
6,6-dimethyl-4-oxo-5,6-dihydro-4H-pyran-2-carboxylic acid (20.5 mg, 0.12 mmol) was dissolved in tetrahydrofuran (1 mL) and stirred at room temperature for one hour with oxalyl chloride (10.5 μL, 0.12 mmol) added. The compound of Reference Example 001 (21.7 mg, 0.10 mmol), pyridine (24.3 μL, 0.30 mmol) and 4-dimethylaminopyridine (2.4 mg, 0.02 mmol) were added to this reaction liquid and stirred at room temperature overnight. Distilled water (1 mL) and 2M aqueous sodium hydroxide solution (0. 25 mL) were added to the reaction liquid and extracted with methylene chloride (4 mL). After washed with 4M hydrochloric acid (3 mL) x 2, distilled water (3 mL), 5% potassium carbonate aqueous solution (3 mL) and then with distilled water (3 mL), the organic layer was concentrated under reduced pressure, and the title compound was obtained (23.3 mg, yield 63%).

Example 035: Synthesis of (2S)-2-methyl-1-(7-thiophen-2-yl-[1,2,4]tri-azolo[1,5-a]pyrimidin-2-ylcarbamoyl) propyl carbamic acid tert-butyl ester (example of Reaction Formula A)
N-(tert-butoxycarbonyl)-L-valine (21.7 mg, 0.10 mmol) and TFFH (25.0mg, 0.10 mmol) were dissolve in tetrahydrofuran (1 mL), added with N,N-diisopropylethylamine (34.8 μL, 0.20 mmol) and stirred at room temperature for one hour to prepared an acid fluoride. The compound of Reference Example 001 (21.7 mg, 0.10 mmol) was suspended in tetrahydrofuran (1 mL) and added with hexamethyldisilazane lithium salt (1M tetrahydrofuran solution, 0.2 mL) and stirred at room temperature for 10 minutes. The acid fluoride solution prepared above was added to this reaction liquid and stirred at room temperature for 15 minutes. After added with distilled water (1 mL) and stirred for one hour, the mixture was diluted with methylene chloride (4 mL) and methanol (1 mL), and washed with 4M hydrochloric acid (3 mL x 2), distilled water (3 mL), 5% potassium carbonate aqueous solution (3 mL x 2) and then with distilled water (3 mL). The solvent was distilled off under reduced pressure, and the title compound was obtained (14.8 mg, yield 36%).

Example 040: Synthesis of ((S)-2-amino-3-methyl-N-(7-thiophen-2-yl-[1,2,4]triazolo[1,5-a]pyrimidin-2-yl)butylamide

To the compound (13.0 mg, 0. 031 mmol) of Example 035, anisole (50 μL) and trifluoroacetic acid (1 mL) were added and stirred at room temperature for 40 minutes. Methanol (1. 5 mL) was added to the reaction liquid and the mixture was concentrated under reduced pressure. After added with 1M hydrochloric acid aqueous solution (1 mL) and methanol (2 mL) and concentrated under reduced pressure, the mixture was dissolved with distilled water (3 mL) and methanol (2 mL) added. Methanol was distilled off under reduced pressure, and the obtained aqueous solution was freeze-dried and hydrochloride salt (11.1 mg, quantitative) of the title compound was obtained.

Example 044: Synthesis of (S)-2-amino-N-(7-thiophen-2-yl-[1,2,4]triazolo[1,5-a]pyrimidin-2-yl)butylamide

To the compound (15.7 mg, 0. 039 mmol) of Example 047, anisole (100 μL) and trifluoroacetic acid (1 mL) were added and stirred at room temperature for one hour. After concentrated under reduced pressure, the reaction liquid was washed with isopropyl ether. After dissolved with 1M hydrochloric acid aqueous solution (1 mL), the residue was concentrated under reduced pressure again. Distilled water was added and the obtained aqueous solution was freeze-dried and hydrochloride salt (13.5 mg, quantitative) of the title compound was obtained.

Example 047: Synthesis of (S)-(1-(7-thiophen-2-yl-[1,2,4] triazolo[1,5-a]-pyrimidin-2-ylcarbamoyl)propyl)carbamic acid tert-butyl ester (example of Reaction Formula A)

(S)-2-(tert-butoxycarbonylamino)butyric acid (40.6 mg, 0.20mmol) and TFFH (50.0mg, 0.20 mmol) were dissolve in tetrahydrofuran (1 mL), added with N,N-diisopropylethylamine (69.7 μL, 0.40 mmol) and stirred at room temperature for one hour to prepared an acid fluoride. The compound of Reference Example 001 (43.4 mg, 0.20 mmol) was suspended in tetrahydrofuran (2 mL) and added with hexamethyldisilazane lithium salt (1M tetrahydrofuran solution, 0.5 mL) and stirred at room temperature for 10 minutes. The acid fluoride solution prepared above was added to this reaction liquid and stirred at room temperature for 10 minutes. After added with N,N-dimethylethylenediamine (44 μL) and stirred for one hour, the mixture was diluted with methylene chloride (4 mL) and methanol (1 mL), and washed with 4M hydrochloric acid (3 mL x 2), distilled water (3 mL), 5% potassium carbonate aqueous solution (3 mL x 2) and then with distilled water (3 mL). The solvent was distilled off under reduced pressure, and the title compound was obtained (23.6 mg, yield 29%).

Example 051: Synthesis of furan-2-carboxylic acid (7-(4-methoxyphenyl)-[1,2,4]triazolo[1,5-a]pyrimidin-2-yl) amide (example of Reaction Formula A)

The compound of Reference Example 005 (96.5 mg, 0.40 mmol) was suspended in tetrahydrofuran (5 mL) and added with hexamethyldisilazane lithium salt (1M tetrahydrofuran solution, 0.80 mL) and stirred at room temperature for 5 minutes. Furan-2-carboxylic acid chloride (40.0 μL, 0. 41 mmol) was added to this reaction liquid and stirred at room temperature for 10 minutes. After added with N,N-dimethylethylenediamine (0.08 mL) and stirred for 20 minutes, the mixture was diluted with methylene chloride (12 mL). The mixture was washed with 4M hydrochloric acid (4 mL x 3), distilled water (4 mL), 5% potassium carbonate aqueous solution (4 mL) and distilled water (4 mL) in this order. The organic layer was concentrated under reduced pressure, and the title compound was obtained (72.3 mg, yield 54%).

$^1$H-NMR (DMSO-$d_6$, ppm): 3.90 (3H, s), 6.73 (1H, dd, J = 1.7, 3.5Hz).7.20(2H, m), 7.59(1H, d, J = 4.9Hz), 7.60(1H, m), 7.99(2H, m), 8.38(1H, m), 8.80(1H, d, J = 4.9 Hz), 11.35(1H, s)

Example 054: Synthesis of 3-benzenesulfonyl-N-(7-(3-methoxyphenyl)-[1,2,4]triazolo[1,5-a]pyrimidin-2-yl)propionamide (example of Reaction Formula B)

3-(phenylsulfonyl) propionic acid (129 mg, 0.60 mmol) was suspended in methylene chloride (2 mL) and added with diisopropyl carbodiimide (47 μL, 0.30 mmol) and stirred at room temperature for 30 minutes to prepare an acid anhydride. The compound of Reference Example 8 (30.0 mg, 0.12 mmol) was dissolves in 1,3-dimethylimidazolidin-2-one (0.6 mL), added with the acid anhydride solution prepared above, and stirred at 100°C for 2.5 hours. Distilled water (13 mL), sodium chloride (0.5 g) and 6M aqueous sodium hydroxide (0.5 mmol) were added to the reaction liquid and the centrifugation supernatant was removed. The residue was suspended and washed with distilled water (10 mL x 2) and methylene chloride (8 mL x 2) successively and the title compound was obtained (19.2 mg, yield 37%).

$^1$H-NMR($CDCl_3$, ppm): 2.88 (2H, m), 3.63 (2H, t, J = 7.6 Hz), 3. 87 (3H, s), 7.24 (1H, m) .7.5-8.0(9H, overlapped), 8.82 (1H, d, J = 4.8 Hz), 11.17(1H, brs).

Example 056: Synthesis of 3-benzenesulfonyl-N-(7-(4-methoxyphenyl)-[1,2,4]triazolo[1,5-a]pyrimidin-2-yl)propionamide (example of Reaction Formula B)

3-(phenylsulfonyl) propionic acid (171.4 mg, 0.80 mmol) was suspended in methylene chloride (3 mL) and added with diisopropyl carbodiimide (62.6 μL, 0.40 mmol) and stirred at room temperature for 30 minutes to prepare an acid anhydride. The compound of Reference Example 005 (48.3 mg, 0.20 mmol) was dissolves in 1,3-dimethylimidazolidin-2-one

(0.8 mL), added with the acid anhydride solution prepared above, and stirred at 100°C for 3 hours. Distilled water (10 mL) was added to the reaction liquid and the centrifugation supernatant was removed. The residue was suspended and washed with 5% potassium carbonate aqueous solution (x 2), distilled water, 4M hydrochloric acid water (x 3) and distilled water successively and the title compound was obtained (25.2 mg, yield 29%).

Hereinbelow, structural formulae and physicochemical data of the compounds of Examples 001 to 060 synthesized by using the production processes described above, processes described in Examples and processes apparent to those skilled in the art are shown in Table 6. These were produced using corresponding starting materials and reagents respectively. Compounds having an amino group in the structural formula were obtained by using raw materials in which the amino group was protected with a tert-butoxycarbonyl group and deprotecting according to methods described in the above Example 040 or Example 044.

In the Table, those having designation of "(S)-", "(R)-" or "(S, S)-" in the column of absolute configuration indicate that the compounds are optically active and they are respectively (S)-compound, (R)-compound and (S, S)-compound. Those having designation of "HCl" in the column of salt indicate that the compound was isolated as a hydrochloride.

[Table 6]

| Example Number | Structural Formula | Absolute Configuration | Salt | Production Method | Molecular Weight | ESI-MS m/z |
|---|---|---|---|---|---|---|
| 001 | 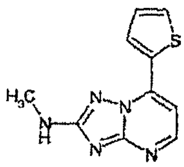 | | | | 231. | 232 |
| 002 | 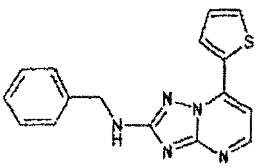 | | | C | 307.4 | 308 |
| 003 | 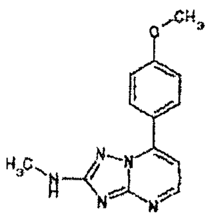 | | | C | 255.3 | 256 |
| 004 | 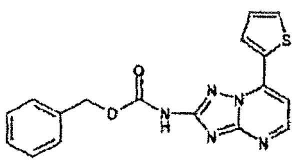 | | | A | 351.4 | 352 |
| 005 | 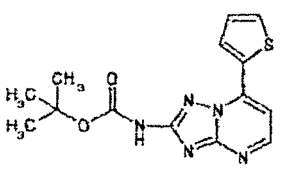 | | | A | 317.4 | 318 |
| 006 | 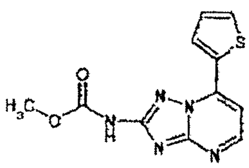 | | | A | 275.3 | 276 |

Table continued

| Example Number | Structural Formula | Absolute Configuration | Salt | Production Method | Molecular Weight | ESI-MS m/z |
|---|---|---|---|---|---|---|
| 007 | 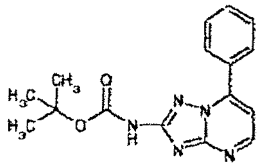 | | | A | 311.3 | 312 |
| 008 | 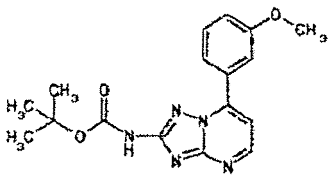 | | | A | 341.4 | 342 |
| 009 | 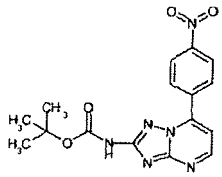 | | | A | 356.3 | 357 |
| 010 | 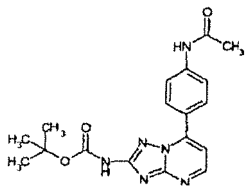 | | | A | 368.4 | 369 |
| 011 | 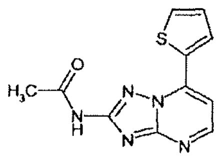 | | | A | 259.3 | 260 |
| 012 | 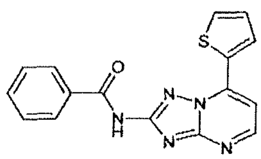 | | | A | 321. 4 | 322 |
| 013 | 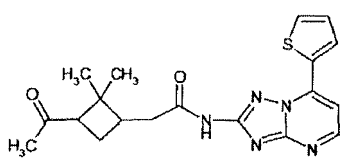 | | | A | 383.5 | 384 |
| 014 | 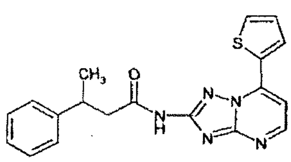 | | | A | 363.4 | 364 |

Table continued

| Example Number | Structural Formula | Absolute Configuration | Salt | Production Method | Molecular Weight | ESI-MS m/z |
|---|---|---|---|---|---|---|
| 015 | 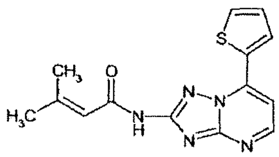 | | | A | 299.4 | 300 |
| 016 | 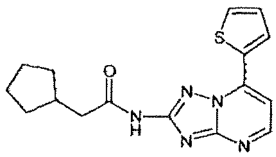 | | | A | 327.4 | 328 |
| 017 | 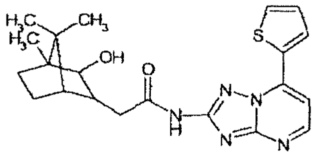 | | | A | 411.5 | 413 |
| 018 | 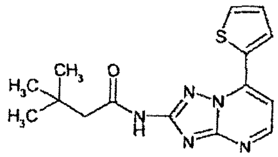 | | | A | 315.4 | 316 |
| 019 | 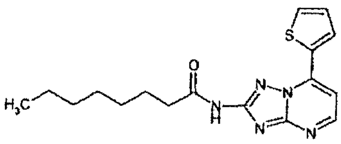 | | | A | 343.5 | 344 |
| 020 | 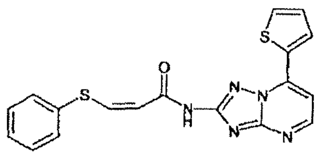 | | | A | 379.5 | 380 |
| 021 | 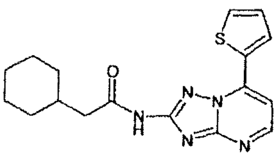 | | | A | 341. 4 | 342 |
| 022 | 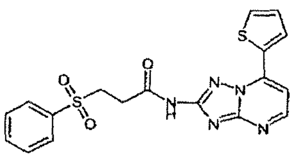 | | | A | 413.5 | 414 |

Table continued

| Example Number | Structural Formula | Absolute Configuration | Salt | Production Method | Molecular Weight | ESI-MS m/z |
|---|---|---|---|---|---|---|
| 023 | 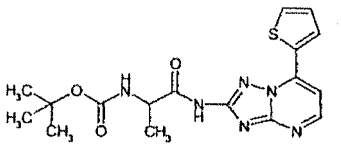 | (R)- | | A | 388. 5 | 389 |
| 024 | 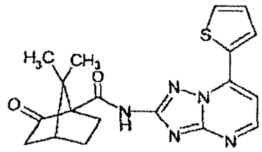 | | | A | 381. 5 | 382 |
| 025 | 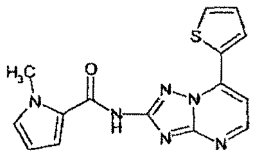 | | | A | 324.4 | 325 |
| 026 | 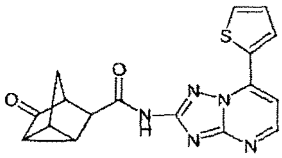 | | | A | 351. 4 | 352 |
| 027 | 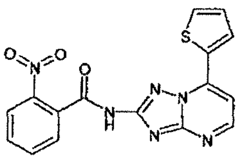 | | | A | 366.4 | 367 |
| 028 | 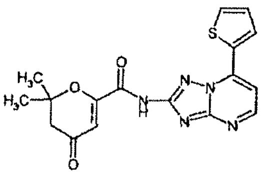 | | | A | 369.4 | 370 |
| 029 | 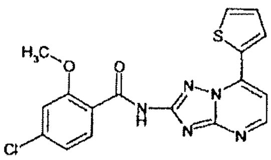 | | | A | 385.8 | 387 |
| 030 | 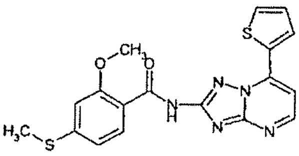 | | | A | 397.5 | 398 |

Table continued

| Example Number | Structural Formula | Absolute Configuration | Salt | Production Method | Molecular Weight | ESI-MS m/z |
|---|---|---|---|---|---|---|
| 031 | 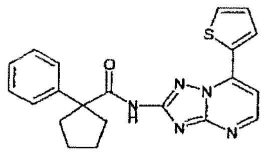 | | | A | 389.5 | 390 |
| 032 | 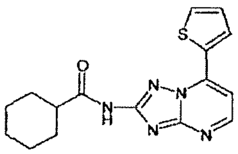 | | | A | 327.4 | 328 |
| 033 | 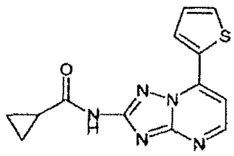 | | | A | 285.3 | 286 |
| 034 | 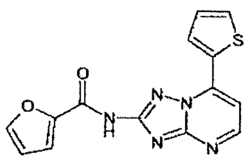 | | | A | 311.3 | 312 |
| 035 | 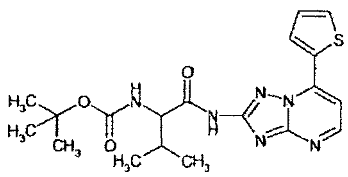 | (S)- | | A | 416.5 | 418 |
| 036 | 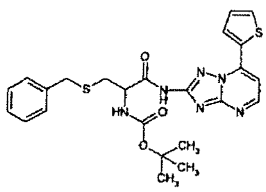 | (S)- | | A | 510.6 | 512 |
| 037 | 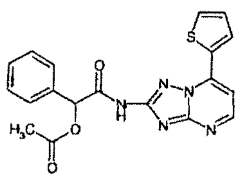 | | | A | 393.4 | 394 |
| 038 | 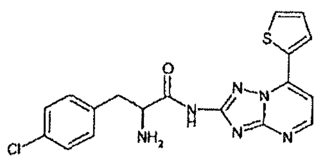 | (S)- | HCl | A | 398.9 | 400 |

Table continued

| Example Number | Structural Formula | Absolute Configuration | Salt | Production Method | Molecular Weight | ESI-MS m/z |
|---|---|---|---|---|---|---|
| 039 | 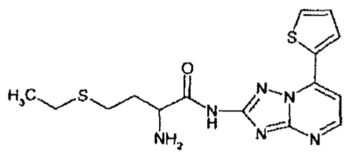 | | HCl | A | 362.5 | 363 |
| 040 | 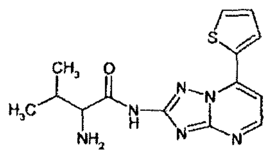 | (S)- | HCl | A | 316.4 | 317 |
| 041 | 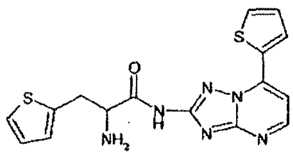 | (RS)- | | A | 370.5 | 371 |
| 042 | 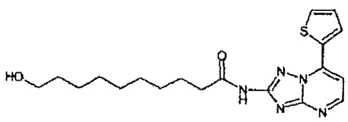 | | | A | 387.5 | 389 |
| 043 | 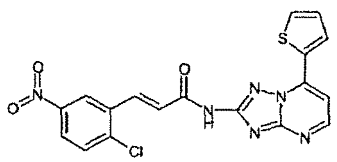 | | | A | 426.8 | 428 |
| 044 | 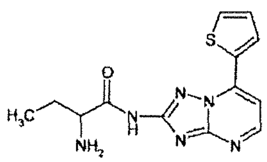 | (S)- | HCl | A | 302. 4 | 303 |
| 045 | 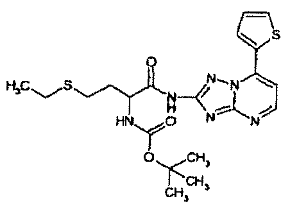 | | | A | 462.6 | 464 |
| 046 | 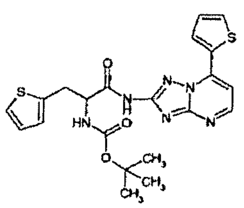 | | | A | 470.6 | 472 |

Table continued

| Example Number | Structural Formula | Absolute Configuration | Salt | Production Method | Molecular Weight | ESI-MS m/z |
|---|---|---|---|---|---|---|
| 047 | 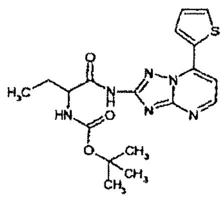 | (S)- | | A | 402. 5 | 403 |
| 048 | 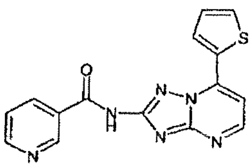 | | | A | 322.4 | 323 |
| 049 | 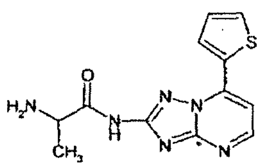 | (R)- | HCl | A | 288.3 | 289 |
| 050 | 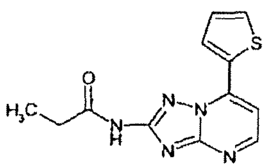 | | | A | 273.3 | 274 |
| 051 | 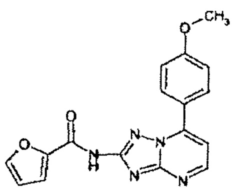 | | | A | 335.3 | 336 |
| 052 | 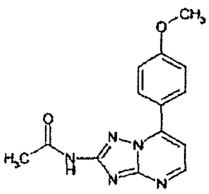 | | | A | 283.3 | 284 |
| 053 | 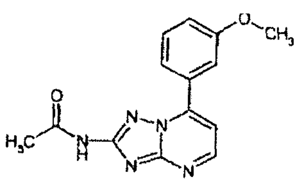 | | | A | 283.3 | 284 |
| 054 | 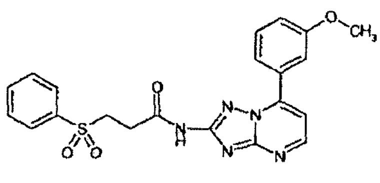 | | | B | 437.5 | 438 |

Table continued

| Example Number | Structural Formula | Absolute Configuration | Salt | Production Method | Molecular Weight | ESI-MS m/z |
|---|---|---|---|---|---|---|
| 055 | 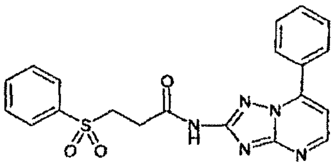 | | | B | 407.5 | 408 |
| 056 | 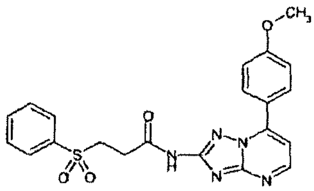 | | | B | 437.5 | 438 |
| 057 | 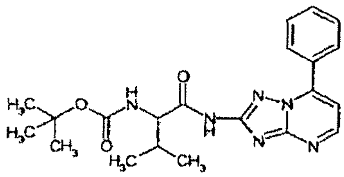 | (S)- | | A | 410.5 | 411 |
| 058 | 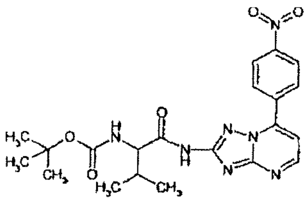 | (S)- | | A | 455.5 | 456 |
| 059 | 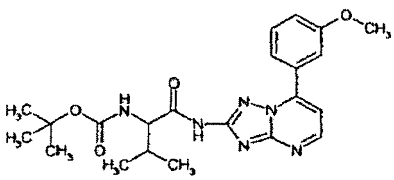 | (S)- | | A | 440.5 | 442 |
| 060 | 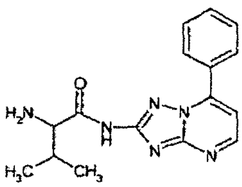 | (S)- | HCl | A | 310.4 | 311 |

**Claims**

**1.** A drug comprising as an active ingredient a substituted 2-amino-[1,2,4]triazolo[1,5-a]pyrimidine derivative represented by the general formula (I):

[Formula 1]

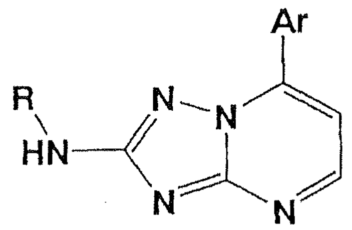

(I)

wherein Ar represents a phenyl group which may have a substituent or a 5- or 6-membered aromatic heterocyclic group which contains one hetero atom and may have a substituent; and
R represents a (C1-C6) alkyl group which may be substituted with a substituent selected from the substituent group consisting of a halogeno group, a cyano group, a nitro group, a hydroxyl group, a (C1-C6) alkoxyl group, a benzyloxy group, a phenyl group and a 5- or 6-membered aromatic heterocyclic group which contains one hetero atom, a (C2-C7) alkoxycarbonyl group which may be substituted with a substituent, a (C1-C13) aliphatic acyl group which may have 1 to 3 same or different substituents, an amino acid group in which the N-terminal may be protected or a 3- to 7-membered cyclic acyl group which may have 1 to 3 same or different substituents, or a pharmaceutically acceptable salt thereof.

2. The drug comprising as an active ingredient a substituted 2-amino-[1,2,4]triazolo[1,5-a]pyrimidine derivative or a pharmaceutically acceptable salt thereof according to Claim 1, wherein Ar of the general formula (I) is a phenyl group which may be substituted with 1 to 3 same or different substituents selected from the substituent group (A) consisting of a halogeno group, a hydroxyl group, a (C1-C6) alkyl group, a cyano group, a nitro group, a (C1-C6) alkoxyl group, a benzyloxy group, an amino group, a (C1-C7) acylamino group and a methylenedioxy group, or a 5- or 6-membered aromatic heterocyclic group which contains one hetero atom independently selected from N, O and S and may be substituted with 1 to 3 same or different substituents selected from the substituent group (A); and
R represents a (C1-C6) alkyl group which may be substituted with one substituent selected from the substituent group consisting of a halogeno group, a hydroxyl group, a (C1-C6) alkoxyl group, a phenyl group and a 5- or 6-membered aromatic heterocyclic group which contains one hetero atom;
a (C2-C7) alkoxycarbonyl group which may be substituted with one substituent selected from the substituent group consisting of a phenyl group, a methoxyphenyl group and a 5- or 6-membered aromatic heterocyclic group which contains one hetero atom;
a (C1-C13) aliphatic acyl group which may have 1 to 3 same or different substituents selected from the substituent group consisting of a halogeno group, a hydroxyl group, an oxo group, a (C1-C6) alkoxyl group, a (C1-C7) acyl group, a (C1-C7) acyloxy group, a trifluoromethyl group, a trifluoromethoxy group, a cyano group, a nitro group, a (C1-C6) alkylsulfanyl group, a benzylsulfanyl group, an arylsulfanyl group, a (C1-C6) alkylsulfonyl group, an arylsulfonyl group, a (C1-C6) alkoxycarbonyl group, an amino group, a (C1-C6) alkylamino group, a di(C1-C6) alkylamino group, a (C1-C7) acylamino group, a (C1-C6) alkoxycarbonylamino group, a benzyloxycarbonylamino group, a phenyl group, wherein the phenyl group may be substituted with 1 to 3 substituents selected from the substituent group consisting of a halogeno group, a (C1-C6) alkyl group, a trifluoromethyl group, a cyano group, a nitro group, a (C1-C6) alkoxyl group, a benzyloxy group, an amino group and a di(C1-C6) alkylamino group, or a 5- or 6-membered saturated or unsaturated heterocyclic group which contains 1 to 4 hetero atoms independently selected from N, O and S and may be substituted with 1 to 3 (C1-C6) alkyl groups;
an α-amino acid group which may be protected at the N-terminal; or
a cyclic acyl group represented by Z-CO-, wherein Z represents an aromatic hydrocarbon group which may have 1 to 3 same or different substituents selected from the substituent group (B) consisting of a (C1-C6) alkyl group, a halogeno group, a hydroxyl group, an oxo group, a trifluoromethyl group, a cyano group, a nitro group, a (C1-C6) alkoxyl group, a benzyloxy group, a (C1-C6) alkylsulfanyl group, a (C1-C6) alkoxycarbonyl group, an amino group, a di(C1-C6) alkylamino group, a (C1-C7) acylamino group and a methylenedioxy group, or a 5- or 6-membered saturated or unsaturated heterocyclic group which contains 1 to 4 hetero atoms independently selected from N, O and S and may be substituted with 1 to 3 same or different substituents selected from the substituent group (B).

3. The drug comprising as an active ingredient a substituted 2-amino-[1,2,4]triazolo[1,5-a]pyrimidine derivative or a pharmaceutically acceptable salt thereof according to Claim 2, wherein Ar of the general formula (I) is a phenyl

group which may be substituted with 1 to 3 same or different substituents selected from the substituent group consisting of a fluoro group, a chloro group, a hydroxyl group, a methoxy group, an ethoxy group, an isopropoxy group, an amino group, an acetylamino group, a propionylamino group and a methylenedioxy group, an unsubstituted thiophen-2-yl group or an unsubstituted thiophen-3-yl group; and
R is a (C2-C4) aliphatic acyl group which may have one substituent selected from the substituent group consisting of a (C1-C6) alkylsulfanyl group, an arylsulfanyl group, a (C1-C6) alkylsulfonyl group and an arylsulfonyl group, or an α-amino acid group represented by the formula (II):

[Formula 2]

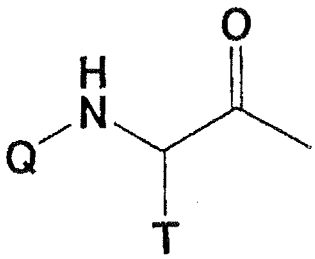

(II)

wherein T represents a hydrogen atom, a methyl group, an ethyl group, a propyl group or an isopropyl group; Q represents a hydrogen atom, a (C1-C7) acyl group or a (C1-C6) alkoxycarbonyl group.

**4.** The drug comprising as an active ingredient a substituted 2-amino-[1,2,4]triazolo[1,5-a]pyrimidine derivative or a pharmaceutically acceptable salt thereof according to Claim 3, wherein Ar of the general formula (I) is a 3-methoxyphenyl group, a 4-methoxyphenyl group or a thiophen-2-yl group; and
R is a 3-methanesulfonylpropionyl group, a 3-benzenesulfonylpropionyl group, a 2-aminopropionyl group, 2-aminobutyryl group, a 2-amino-3-methylbutyryl group, a 2-acetylaminopropionyl group, a 2-acetylamino-3-methylbutyryl group, a 2-(tert-butoxycarbonyl)aminopropionyl group or a 2-(tert-butoxycarbonyl)amino-3-methylbutyryl group.

**5.** The drug comprising as an active ingredient a substituted 2-amino-[1,2,4]triazolo[1,5-a]pyrimidine derivative or a pharmaceutically acceptable salt thereof according to Claim 4, wherein R is an (S)-2-aminopropionyl group, an (S)-2-aminobutyryl group, an (S)-2-amino-3-methylbutyryl group, an (S)-2-acetylaminopropionyl group, an (S)-2-acetylamino-3-methylbutyryl group, an (S)-2-(tert-butoxycarbonyl)aminopropionyl group or an (S)-2-(tert-butoxycarbonyl)amino-3-methylbutyryl group.

**6.** The drug according to Claim 1, wherein the substituted 2-amino-[1,2,4]triazolo[1,5-a]pyrimidine derivative represented by the general formula (I) is 3-benzenesulfonyl-N-(7-thiophen-2-yl-[1,2,4]triazolo[1,5-a]pyrimidin-2-yl)propionamide, 3-benzenesulfonyl-N-(7-(4-methoxyphenyl)-[1,2,4]triazolo[1,5-a]pyrimidin-2-yl)propionamide, (S)-2-(tert-butoxycarbonyl)amino-N-(7-thiophen-2-yl-[1,2,4]triazolo[1,5-a]pyrimidin-2-yl)butylamide, (S)-2-amino-N-(7-thiophen-2-yl-[1,2,4]triazolo[1,5-a]pyrimidin-2-yl)butylamide or (S)-2-amino-3-methyl-N-(7-thiophen-2-yl-[1,2,4]triazolo[1,5-a]pyrimidin-2-yl)butylamide.

**7.** An antigen presentation inhibitor comprising as an active ingredient a substituted 2-amino-[1,2,4]triazolo[1,5-a]pyrimidine derivative or a pharmaceutically acceptable salt thereof according to any one of Claims 1 to 6.

**8.** An immunosuppresant or an immunotolerance inducer comprising as an active ingredient a substituted 2-amino-[1,2,4]triazolo[1,5-a]pyrimidine derivative or a pharmaceutically acceptable salt thereof according to any one of Claims 1 to 6.

**9.** A therapeutic agent or a prophylactic agent for transplant rejection reaction or graft versus host reaction diseases comprising as an active ingredient a substituted 2-amino-[1,2,4]triazolo[1,5-a]pyrimidine derivative or a pharmaceutically acceptable salt thereof according to any one of Claims 1 to 6.

**10.** A therapeutic agent or a prophylactic agent for autoimmune diseases comprising as an active ingredient a substituted 2-amino-[1,2,4]triazolo[1,5-a]pyrimidine derivative or a pharmaceutically acceptable salt thereof according to any one of Claims 1 to 6.

**11.** A therapeutic agent or a prophylactic agent for rheumatoid arthritis, multiple sclerosis, systemic lupus erythematosus, discoid lupus erythematosus, Sjogren's syndrome, Crohn's disease, colitis ulcerosa, idiopathic thrombocytopenia, aplastic anemia, autoimmune hepatitis, insulin-dependent diabetes, myasthenia gravis, polymyositis, scleroderma, mixed connective tissue disease, ankylosing spondylitis or chronic thyroiditis comprising as an active ingredient a substituted 2-amino-[1,2,4]triazolo[1,5-a]pyrimidine derivative or a pharmaceutically acceptable salt thereof according to any one of Claims 1 to 6.

**12.** A therapeutic agent or a prophylactic agent for allergic diseases comprising as an active ingredient a substituted 2-amino-[1,2,4]triazolo[1,5-a]pyrimidine derivative or a pharmaceutically acceptable salt thereof according to any one of Claims 1 to 6.

**13.** A therapeutic agent or a prophylactic agent for atopic dermatosis, pollinosis, contact hypersensitivity, asthma, psoriasis or anaphylaxis comprising as an active ingredient a substituted 2-amino-[1,2,4]triazolo[1,5-a]pyrimidine derivative or a pharmaceutically acceptable salt thereof according to any one of Claims 1 to 6.

**14.** A therapeutic agent or a prophylactic agent for inflammatory diseases comprising as an active ingredient a substituted 2-amino-[1,2,4]triazolo[1,5-a]pyrimidine derivative or a pharmaceutically acceptable salt thereof according to any one of Claims 1 to 6.

**15.** A therapeutic agent or a prophylactic agent for Behcet's disease, polyarteritis, sarcoidosis, glomerulonephritis, nephrotic syndrome, intractable vasculitis or Wegener's syndrome comprising as an active ingredient a substituted 2-amino-[1,2,4]triazolo[1,5-a]pyrimidine derivative or a pharmaceutically acceptable salt thereof according to any one of Claims 1 to 6.

**16.** A cell proliferation inhibitor antineoplastic drug comprising as an active ingredient a substituted 2-amino-[1,2,4]triazolo[1,5-a]pyrimidine derivative or a pharmaceutically acceptable salt thereof according to any one of Claims 1 to 6.

**17.** An antineoplastic drug comprising as an active ingredient a substituted 2-amino-[1,2,4]triazolo[1,5-a]pyrimidine derivative or a pharmaceutically acceptable salt thereof according to any one of Claims 1 to 6.

**18.** A substituted 2-amino-[1,2,4]triazolo[1,5-a]pyrimidine derivative represented by the general formula (III)

[Formula 3]

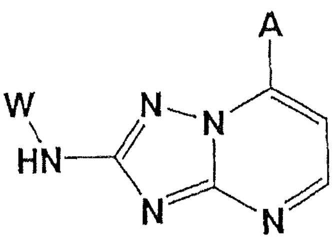

(III)

wherein A represents a phenyl group which may be substituted with 1 to 3 same or different substituents selected from the substituent group (A) consisting of a halogeno group, a hydroxyl group, a (C1-C6) alkyl group, a cyano group, a nitro group, a (C1-C6) alkoxyl group, a benzyloxy group, an amino group, a (C1-C7) acylamino group and a methylenedioxy group or a 5- or 6-membered aromatic heterocyclic group which contains one hetero atom and may be substituted with 1 to 3 same or different substituents selected from the substituent group (A); and
W represents a (C1-C6) alkyl group which may be substituted with one substituent selected from the substituent group consisting of a halogeno group, a hydroxyl group, a (C1-C6) alkoxyl group, a phenyl group and a 5- or 6-membered aromatic heterocyclic group which contains one hetero atom;
a (C2-C7) alkoxycarbonyl group which may be substituted with one substituent selected from the substituent group consisting of a phenyl group, a methoxyphenyl group and a 5- or 6-membered aromatic heterocyclic group which contains one hetero atom;
a (C1-C13) aliphatic acyl group which may have 1 to 3 same or different substituents selected from the substituent group consisting of a halogeno group, a hydroxyl group, an oxo group, a (C1-C6) alkoxyl group, a (C1-C7) acyl

group, a (C1-C7) acyloxy group, a trifluoromethyl group, a trifluoromethoxy group, a cyano group, a nitro group, a (C1-C6) alkylsulfanyl group, a benzylsulfanyl group, an arylsulfanyl group, a (C1-C6) alkylsulfonyl group, an arylsulfonyl group, a (C1-C6) alkoxycarbonyl group, an amino group, a (C1-C6) alkylamino group, a di(C1-C6) alkylamino group, a (C1-C7) acylamino group, a (C1-C6) alkoxycarbonylamino group, a benzyloxycarbonylamino group, a phenyl group, wherein the phenyl group may be substituted with 1 to 3 substituents selected from the substituent group consisting of a halogeno group, a (C1-C6) alkyl group, a trifluoromethyl group, a cyano group, a nitro group, a (C1-C6) alkoxyl group, a benzyloxy group, an amino group, a di(C1-C6) alkylamino group, or a 5- or 6-membered saturated or unsaturated heterocyclic group which contains 1 to 4 hetero atoms independently selected from N, O and S and may be substituted with 1 to 3 (C1-C6) alkyl groups;
an $\alpha$-amino acid group which may be protected at the N-terminal; or
a cyclic acyl group represented by Z-CO-, wherein Z represents an aromatic hydrocarbon group which may have 1 to 3 same or different substituents selected from the substituent group (B) consisting of a (C1-C6) alkyl group, a halogeno group, a hydroxyl group, an oxo group, a trifluoromethyl group, a cyano group, a nitro group, a (C1-C6) alkoxyl group, a benzyloxy group, a (C1-C6) alkylsulfanyl group, a (C1-C6) alkoxycarbonyl group, an amino group, a di(C1-C6) alkylamino group, a (C1-C7) acylamino group and a methylenedioxy group, or a 5- or 6-membered saturated or unsaturated heterocyclic group which contains 1 to 4 hetero atoms independently selected from N, O and S and may be substituted with 1 to 3 same or different substituents selected from the substituent group (B), or a pharmaceutically acceptable salt thereof.

**19.** A substituted 2-amino-[1,2,4]triazolo[1,5-a]pyrimidine derivative which is 3-benzenesulfonyl-N-(7-thiophen-2-yl-[1,2,4]triazolo[1,5-a]pyrimidin-2-yl)propionamide, 3-benzenesulfonyl-N-(7-(4-methoxyphenyl)-[1,2,4]triazolo[1,5-a]pyrimidin-2-yl)propionamide, (S)-2-(tert-butoxycarbonyl)amino-N-(7-thiophen-2-yl-[1,2,4]triazolo[1,5-a]pyrimidin-2-yl)butylamide, (S)-2-amino-N-(7-thiophen-2-yl-[1,2,4]triazolo[1,5-a]pyrimidin-2-yl)butylamide or (S)-2-amino-3-methyl-N-(7-thiophen-2-yl-[1,2,4]triazolo[1,5-a]pyrimidin-2-yl)butylamide, or a pharmaceutically acceptable salt thereof.

**INTERNATIONAL SEARCH REPORT**

International application No.
PCT/JP2004/015245

A. CLASSIFICATION OF SUBJECT MATTER
Int.Cl⁷ C07D487/04, A61K31/519, A61P1/04, 1/16, 3/10, 5/14, 7/04, 7/06, 9/00, 9/06, 13/12, 17/00, 17/04, 17/06, 19/02, 19/04, 19/08, 21/00, 21/04, 25/28, 29/00, 35/00, 37/06, 37/08, 41/00

According to International Patent Classification (IPC) or to both national classification and IPC

B. FIELDS SEARCHED

Minimum documentation searched (classification system followed by classification symbols)
Int.Cl⁷ C07D487/04, A61K31/519

Documentation searched other than minimum documentation to the extent that such documents are included in the fields searched

Electronic data base consulted during the international search (name of data base and, where practicable, search terms used)
CAPLUS(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | WO 2002/002563 A2 (AMERICAN HOME PRODUCTS CORP.), 10 January, 2002 (10.01.02), Full text & CA 2413802 A & US 2002/0068744 A1 & EP 112038 A & JP 16-502691 A | 1,2,16-18 |
| E,A | JP 2004-323458 A (Nippon Kayaku Co., Ltd.), 18 November, 2004 (18.11.04), Claims (Family: none) | 1-19 |
| A | JP 5-148267 A (EGIS Cyogyszergvar), 15 June, 1993 (15.06.93), Claims & CA 2061629 A & GB 2254611 A & US 5478825 A | 1-19 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 December, 2004 (14.12.04) | 28 December, 2004 (28.12.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/015245

C (Continuation). DOCUMENTS CONSIDERED TO BE RELEVANT

| Category* | Citation of document, with indication, where appropriate, of the relevant passages | Relevant to claim No. |
|---|---|---|
| A | JP 7-309872 A (Otsuka Pharmaceutical Factory, Inc.), 28 November, 1995 (28.11.95), Claims; Par. No. [0041] (Family: none) | 1-19 |
| A | JP 56-110620 A (Mochida Pharmaceutical Co., Ltd.), 01 September, 1981 (01.09.81), Claims (Family: none) | 1-19 |

Form PCT/ISA/210 (continuation of second sheet) (January 2004)

**INTERNATIONAL SEARCH REPORT**

International application No.
PCT/JP2004/015245

Continuation of A. CLASSIFICATION OF SUBJECT MATTER
(International Patent Classification (IPC))

Int.Cl$^7$ 43/00//C07D487/04

(According to International Patent Classification (IPC) or to both national classification and IPC)

Form PCT/ISA/210 (extra sheet) (January 2004)